# EUROPEAN PATENT SPECIFICATION

(11) **EP 3 402 443 B1**
(45) Date of publication and mention of the grant of the patent: **06.05.2026**
(21) Application number: 17738974.9
(22) Date of filing: 12.01.2017
(51) Int. Cl.: A61F 2/36, A61L 27/42, A61L 24/00, A61L 26/00, A61L 27/46, A61K 31/12, A61F 2/28, A61F 2/30, A61L 31/12, A61B 17/70, A61L 15/58, A61F 2/44

(54) **DEVICES AND COMPOSITIONS AND METHODS OF USE THEREOF**
VORRICHTUNGEN UND ZUSAMMENSETZUNGEN UND VERFAHREN ZUR VERWENDUNG DAVON
DISPOSITIFS ET COMPOSITIONS ET LEURS PROCÉDÉS D'UTILISATION

(30) Priority: 12.01.2016 US 201662277828 P; 21.10.2016 US 201662411391 P
(43) Date of publication of application: 21.11.2018
(73) Proprietor: RevBio, Inc., Lowell, MA 01854 (US)
(72) Inventor: HESS, Brian, J., Charlestown MA 02129 (US); KAY, George, W., Sharon MA 02067 (US); MARCHENKO, Andrey, Acton MA 01720 (US); BROWN, Michael, C., Braintree MA 02184 (US); KOSH, David, J., Norton MA 02766 (US)
(74) Representative: Abthorpe, Mark
(86) International application number: PCT/US2017/013256
(87) International publication number: WO 2017/123811

(56) References cited:
- US-A- 6 087 553
- US-A1- 2005 217 538
- US-A1- 2011 277 931
- US-A1- 2011 287 067
- US-A1- 2012 082 705
- US-A1- 2012 082 705
- US-A1- 2013 122 057
- US-A1- 2013 122 057
- US-A1- 2013 238 027
- US-A1- 2014 093 549
- US-A1- 2015 072 315
- KAZUHIKO KANDORI ET AL: "Texture and Formation Mechanism of Fibrous Calcium Hydroxyapatite Particles Prepared by Decomposition of Calcium-EDTA Chelates", JOURNAL OF THE AMERICAN CERAMIC SOCIETY., vol. 80, no. 5, 1 May 1997 (1997-05-01), US, pages 1157 - 1164, XP055651185, ISSN: 0002-7820, DOI: 10.1111/j.1151-2916.1997.tb02958.x
- JOHN L MEYER ET AL: "The influence of multidentate organic phosphonates on the crystal growth of hydroxyapatite", CALCIFIED TISSUE INTERNATIONAL, NEW YORK, NY, US, vol. 13, 1 January 1973 (1973-01-01), pages 295 - 303, XP009178766, ISSN: 0171-967X, DOI: 10.1007/BF02015419
- DINARVAND ET AL.: "Polylactide-co-glycolide nanoparticles for controlled delivery of anticancer agents", INTERNATIONAL JOURNAL OF NANOMEDICINE, vol. 6, 26 April 2011 (2011-04-26), pages 877 - 895, XP055168931

## Description

### CLAIM OF PRIORITY

This application claims priority to U.S. Application No. 62/411,391, filed October 21, 2016, and U.S. Application No. 62/277,828, filed January 12, 2016.

### FIELD

Embodiments of the disclosure relate to devices (e.g., patches, plugs, beams, plates, screws, rods, granules, spacers, cages, discs, tape devices, or other shape determined by the geometry or anatomy of the site of application) and methods of use thereof.

### BACKGROUND

The repair of cracks, gaps, fissures, leaks, or other defects in objects can often be a difficult process that is incapable of restoring the original strength, integrity, and appearance to said object, particularly when said objects are subjected to a wet environment. US 2012/082705 A1, for example, describes compositions and methods of their use to adhere a variety of materials together. The compositions include tetra calcium phosphate and an effective amount of a compound that is structurally similar to phosphoserine, and can be mixed with an aqueous solution. Current practices do not seek to replace the missing void material, let alone enhance the strength and structural integrity of the damaged object. Instead, the goal is often to join separated objects or plug a leak, leaving the repaired region weak and vulnerable to repeated damage, especially in a wet environment. As such, there exists a need for new devices and methods for the repair of cracks, fissures, leaks, and other defects in objects, particularly in the surgical and industrial settings. The development of new adhesive compositions that can impart structural integrity to the object and enhance its original strength and performance, e.g., in a wet environment, provide opportunities to cure this need, particularly when used in combination with a setting framework such as a patch, tape, beam, plate, screw, rod, granule, spacer, cage, disc, plug, or other shape determined by the geometry or anatomy of the site of application.

### SUMMARY

This application relates generally to devices and methods of fabrication and use thereof. In one aspect, the disclosure provides an adhesive composition for use in a method of joining separated (e.g., fractured) bones as defined in claim 1, wherein the method comprises forming a device for joining the bones and applying the device at the interposition between the two separated bones. The method may be to treat or heal a subject suffering from a disease or condition. The adhesive composition comprises a multivalent metal salt and an acidic compound. The device comprises a solidified form of the adhesive composition after a reaction between the multivalent metal salt and the acidic compound has completed in the presence of an aqueous medium. The device also comprises a three dimensional fiber network. The device also comprises an additional layer of the adhesive composition in its working state (i.e., after addition of aqueous medium becomes tacky and adhesive) that is coated or impregnated into or onto the surface of the solidified form and allowed to cure during its method of use.

In any and all aspects, the adhesive device may take the form of a patch, plug, beam, plate, screw, rod, granule, spacer, cage, disc, tape device, or other shape determined by the geometry or anatomy of the site of application.

In some embodiments, the three dimensional fiber network material is in the form of a two or three-dimensional grid, lattice, mesh, mat, weave, braid, cloth, fabric, felt, web, open cell foam, sponge, sheet, membrane, cage, or gel. In some embodiments, the mean diameter of the three dimensional fiber network material is from about 25 nanometers to about 500 nanometers or from about 500 nanometers to about 1000 nanometers. In some embodiments, the mean diameter of the three dimensional fiber network material is from 1 micrometer to 100 micrometers, or from 100 micrometers to 500 micrometers, or from 500 micrometers to 1000 micrometers. In some embodiments, the mean diameter of the three dimensional fiber network material is from about 1 millimeter to about 5 millimeters. In some embodiments, the adhesive device comprises more than one three dimensional fiber network, each exhibiting a different mean diameter.

In some embodiments, the three dimensional fiber network comprises a substantially biocompatible or substantially bioresorbable component. In some embodiments, the biocompatible or bioresorbable component comprises poly(L-lactide), poly(D,L-lactide), poly(glycolide), poly(ε-caprolactone), poly(carbonate), poly(ethylene), poly(teramethylglycolic-acid), poly(dioxanone), poly(hydroxybutyrate), poly(hydroxyvalerate), poly(L-lactide-co-glycolide), poly(glycolide-co-trimethylene-carbonate), poly(glycolide-co-caprolactone), poly(glycolide-co-dioxanone-co-trimethylene-carbonate), poly(tetramethylglycolic-acid-co-dioxanone-co-trimethylene-carbonate), poly(glycolide-co-caprolactone-co-L-lactide-co-trimethylene-carbonate), poly(hydroxybutyrate-co-hydroxyvalerate), poly(methyl-methacrylate), a polyamine, a polyimidazole, poly(vinyl-pyrrolidone), chitosan, hyaluronic acid, collagen, gelatin, or a copolymer, derivative, or mixture thereof.

In some embodiments, the three dimensional fiber network comprises a substantially non-resorbable component. In some embodiments, the substantially non-resorbable component comprises silk, nylon, a polyamide, glass, carbon, an aromatic (e.g., polyphenylene vinylene) and/or conjugated (e.g., polyacetylene) polymer, an intrinsically conductive polymer (e.g., polyaniline, polypyrrole, polythiophene), a metal (e.g., calcium, silicon, copper, silver, gold, zinc, iron, titanium, aluminum, cobalt, chromium, tantalum, molybdenum), a metallic alloy (e.g., bronze, brass, steel (e.g., stainless steel), cobalt-chromium), poly(ether ketone), poly(urethane), poly(methyl methacrylate), poly(acrylic acid) or a copolymer, derivative, or mixture thereof.

In some embodiments, the three dimensional fiber network comprises an element that affects its physical or mechanical properties. In some embodiments, the three dimensional fiber network is substantially crosslinked. In some embodiments, the three dimensional fiber network is substantially non-crosslinked. In some embodiments, the three dimensional fiber network is able to withstand or resist a pressure (e.g., a hydrostatic pressure) of at least about 140 kPa (20 psi).

In some embodiments, the three dimensional fiber network further comprises a mesh component (e.g., a membrane, sheet, coating, fiber, rod, plate, or strut). In some embodiments, the mesh component is permeable, e.g., and allows for movement of particulates or other substances. In some embodiments, the mesh component is substantially impermeable, e.g., and does not allow for movement of particulates or other substances. In some embodiments, the mesh component is substantially compliant or deformable, e.g., elastic, plastic, or able to conform to the shape and sizes of the objects to which it is applied. In some embodiments the compliance of the mesh component is isotropic. In some components the compliance of the mesh component is anisotropic, i.e., the mechanical properties of the material are not equal in different dimensions.

In some embodiments, the mesh component comprises a substantially biocompatible or substantially bioresorbable component. In some embodiments, the biocompatible or bioresorbable component comprises poly(L-lactide), poly(D,L-lactide), poly(glycolide), poly(ε-caprolactone), poly(carbonate), poly(ethylene), poly(teramethylglycolic-acid), poly(dioxanone), poly(hydroxybutyrate), poly(hydroxyvalerate), poly(L-lactide-co-glycolide), poly(glycolide-co-trimethylene-carbonate), poly(glycolide-co-caprolactone), poly(glycolide-co-dioxanone-co-trimethylene-carbonate), poly(tetramethylglycolic-acid-co-dioxanone-co-trimethylene-carbonate), poly(glycolide-co-caprolactone-co-L-lactide-co-trimethylene-carbonate), poly(hydroxybutyrate-co-hydroxyvalerate), poly(methyl-methacrylate), a polyamine, a polyimidazole, poly(vinyl-pyrrolidone), chitosan, hyaluronic acid, collagen, gelatin, or a copolymer, derivative, or mixture thereof.

In some embodiments, the mesh component comprises a substantially non-resorbable component. In some embodiments, the substantially non-resorbable component comprises silk, nylon, a polyamide, glass, carbon, an aromatic (e.g., polyphenylene vinylene) and/or conjugated (e.g., polyacetylene) polymer, an intrinsically conductive polymer (e.g., polyaniline, polypyrrole, polythiophene), a metal (e.g., calcium, silicon, copper, silver, gold, zinc, iron, titanium, aluminum, cobalt, chromium, tantalum, molybdenum), a metallic alloy (e.g., bronze, brass, steel (e.g., stainless steel), cobalt-chromium), poly(ether ketone), poly(ethylene), poly(urethane), poly(methyl methacrylate), or poly(acrylic acid) or a copolymer, derivative, or mixture thereof.

In some embodiments, the mesh component is able to withstand or resist a pressure (e.g., a hydrostatic pressure) of at least about 140 kPa (20 psi).

The adhesive composition comprises a multivalent metal salt and an acidic compound. The acidic compound comprises a compound of Formula (I) or a salt thereof: 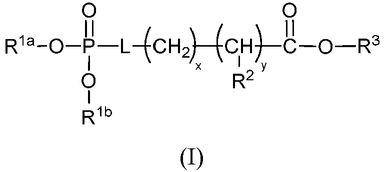 wherein L is O, S, NH, or CH₂; each of R^{1a} and R^{1b} is independently H, optionally substituted alkyl, or optionally substituted aryl; R² is H, NR^{4a}R^{4b}, C(O)R⁵, or C(O)OR⁵; R³ is H, optionally substituted alkyl, or optionally substituted aryl; each of R^{4a} and R^{4b} is independently H, C(O)R⁶, or optionally substituted alkyl; R⁵ is H, optionally substituted alkyl, or optionally substituted aryl; R⁶ is optionally substituted alkyl or optionally substituted aryl; and each of x and y is independently 0, 1, 2, or 3.

In some embodiments, L is O or S. In some embodiments, L is O. In some embodiments, each of R^{1a} and R^{1b} is independently H. In some embodiments, L is O, and each of R^{1a} and R^{1b} is H. In some embodiments, R² is H, NR^{4a}R^{4b}, or C(O)R⁵. In some embodiments, R² is NR^{4a}R^{4b}. In some embodiments, R² is NR^{4a}R^{4b} and each of R^{4a} and R^{4b} is independently H. In some embodiments, L is O, each of R^{1a} and R^{1b} is independently H, R² is NR^{4a}R^{4b}, and each of R^{4a} and R^{4b} is independently H. In some embodiments, R³ is H. In some embodiments, L is O, each of R^{1a} and R^{1b} is independently H, R² is NR^{4a}R^{4b}, each of R^{4a} and R^{4b} is independently H, and R³ is H. In some embodiments, each of x and y is independently 0 or 1. In some embodiments, each of x and y is independently 1. In some embodiments, L is O, each of R^{1a} and R^{1b} is independently H, R² is NR^{4a}R^{4b}, each of R^{4a} and R^{4b} is independently H, R³ is H, and each of x and y is 1. In some embodiments, the compound of Formula (I) is phosphoserine.

In some embodiments, the multivalent metal salt comprises calcium. In some embodiments, the multivalent metal salt comprises calcium and phosphate. In some embodiments, the multivalent metal salt comprises tetracalcium phosphate. In some embodiments, the multivalent metal salt comprises tricalcium phosphate. In some embodiments, the tricalcium phosphate comprises either alpha tricalcium phosphate or beta tricalcium phosphate.

In some embodiments, the multivalent metal salt comprises an oxide. In some embodiments, the multivalent metal salt comprises calcium oxide. In some embodiments, the composition comprises a combination of multivalent metal salts (e.g., tricalcium phosphate and calcium oxide). In some embodiments, the composition does not contain tetracalcium phosphate. In some embodiments, the multivalent metal salt (e.g., tetracalcium phosphate, tricalcium phosphate, calcium oxide or a combination thereof) is present in an amount from about 15% to about 85% weight by weight (w/w) of the total adhesive composition.

In the present disclosure, the multivalent metal salt (*e.g*., calcium phosphates, calcium oxide or combinations thereof) may with the acidic compound to form an adhesive composition that is self-setting and solidifies when combined with an aqueous medium. In some embodiments, the adhesive composition further comprises an aqueous medium. In some embodiments, the aqueous medium comprises water (*e.g*., sterile water), oral fluids (e.g., saliva, sulcular fluids, mucus, blood, or blood mixtures) buffers (*e.g*., sodium phosphate, potassium phosphate, or saline), blood, blood-based solutions (*e.g.,* plasma, serum, bone marrow), spinal fluid, dental pulp, cell-based solutions (*e.g,* solutions comprising fibroblasts, platelets, odontoblasts, erythrocytes, leukocytes, stem cells (*e.g*., mesenchymal stem cells) histiocytes, macrophages, mast cells, or plasma cells), environmental water (*e.g.*, marine, fluvial or lacustrine (i.e., derived from the ocean or freshwater sources, *e.g*., bays, lakes, streams, rivers, marshes, or ponds)), industrial process fluid, waste water (e.g., gray water or black water), or combinations thereof. In some embodiments, the aqueous medium comprises saliva, serum or blood.

In some embodiments, the adhesive composition does not comprise an aqueous medium (e.g., water).

In some embodiments, the multivalent metal salt is initially provided as granules or a powder. This powder may exhibit a mean particle size of about 0.001 to about 0.750 mm, about 0.005 to about 0.150 mm, about 0.250 to about 0.750 mm, 0.25 to about 0.500, 0.015 to about 0.050 mm, about 0.015 to about 0.025 mm, about 0.020 to about 0.060 mm, about 0.020 to about 0.040 mm, about 0.040 to about 0.100 mm, about 0.040 to about 0.060 mm, about 0.060 to about 0.150 mm, or about 0.060 to about 0.125 mm. The mean particle size may be bi-modal to include any combination of mean particle sizes as previously described. These granules may exhibit a mean granule size of about 0.050 mm to about 5 mm, about 0.100 to about 1.500 mm, about 0.125 to 1.000 mm, 0.125 to 0.500 mm, about 0.125 to 0.250 mm, about 0.250 to 0.750 mm, about 0.250 to 0.500 mm, about 0.500 to 1.00 mm, about 0.500 to 0.750 mm. The mean granule size may be multi-modal to include any combination of mean granule sizes as previously described. In some embodiments, varying sizes of said powder or granules may be used in the adhesive composition.

In some embodiments, the adhesive composition further comprises an additive or a plurality of said additives present in an amount from about 1% to about 95% weight by weight (w/w) of the total adhesive composition. An additive may be used to impart additional functionality to the composition of the disclosure, such as improving or affecting the handling, texture, porosity, durability, elasticity, flexibility, stiffness, toughness, strength, or resorption rate of the material, or to provide additional cosmetic or medical properties.

Exemplary additives may include a metal (*e.g*. copper, silver, gold, iron, titanium, aluminum, cobalt, chromium, tantalum), a metallic alloy (*e.g*. bronze, brass, stainless steel, cobalt-chromium), a salt comprising calcium, sodium, barium, strontium, lithium, potassium, magnesium a phosphate, an oxide, hydroxide, iodide, a sulfate, a carbonate, fluoride, or chloride (*e.g.,* calcium phosphates (*e.g.,* dicalcium phosphate, monocalcium phosphate, beta tricalcium phosphate, hydroxyapatite, calcium depleted hydroxyapatites, alpha tricalcium phosphate), calcium oxide, calcium sulfate, calcium carbonate, calcium bicarbonate, calcium iodide, barium sulfate, barium phosphate, sodium phosphate, sodium carbonate, sodium bicarbonate, sodium chloride, magnesium phosphate, potassium chloride), a pore forming agent (*e.g*., through release of gas (e.g., calcium carbonate, sodium carbonate, sodium bicarbonate) or through dissolution of a solid (*e.g.,* sodium chloride, potassium chloride)), a humectant (*e.g*., sorbitol, or another hygroscopic compound), reducing or oxidizing agents, rust inhibitors, a polymeric alcohol (*e.g.,* polyethylene glycol), a filler, a formulation base, a viscosity modifier (*e.g.,* polyol (*e.g.,* glycerol, mannitol, sorbitol, trehalose, lactose, glucose, fructose, or sucrose)), an abrasive , a coloring agent (*e.g.,* a dye, pigment, or opacifier), a flavoring agent (*e.g.,* a sweetener), a medication that acts locally (*e.g.,* an anesthetic, coagulant, clotting factor, chemotactic agent, and an agent inducing phenotypic change in local cells or tissues), a medication that acts systemically (*e.g*., an analgesic, anticoagulant, hormone, vitamin, pain reliever, anti-inflammatory agent, chemotactic agent, or agent inducing phenotypic change in local cells or tissues), an antimicrobial agent (*e.g.,* an antibacterial, antiviral, antifungal agent), an antifouling agent (e.g., copper, silver, or other transition metal salts) or a combination thereof. In some embodiments, the additive comprises a polymer. The biologically active substances (*e.g.*, medicines, drugs) in the categories above might include active substances or precursors, which become biologically active upon modification after interaction with the surrounding environment. The biologically active substances might include a stem cell (*e.g.,* an adult, induced pluripotent, or mesenchymal stem cell) or bone tissue components (e.g., autograft, allograft, xenograft, bone marrow). The substances might be synthetic, semisynthetic, or biologically derived (e.g., peptides, proteins, or small molecules). The substances might include, but not be limited to, an anti-inflammatory compound (e.g., a steroid, nonsteroidal anti-inflammatory drug, or cyclooxygenase inhibitor), a complement protein, a bone morphogenic factor or protein, a hormone active locally or systemically (e.g., parathyroid hormone, calcitonin, or prostaglandin, or derivative), or other small molecule (*e.g.,* a calciferol, secosteroids).

In some embodiments, the additive is a solidified form of the adhesive composition. In some embodiments, the additive comprises a powder or granule, or is in the form of a powder or granules. In some embodiments, the solidified form of the adhesive composition is a product from the reaction of a multivalent metal salt and an acidic compound in an aqueous medium. In some embodiments, the solidified form of the adhesive composition comprises hydroxyapatite, tetracalcium phosphate, tricalcium phosphate, or any other calcium salt, e.g., as a byproduct from the reaction of a calcium phosphate metal salt, an acidic compound, and an additive or a plurality of additives in an aqueous medium. In some embodiments, the solidified form of the adhesive composition comprises hydroxyapatite, tetracalcium phosphate, tricalcium phosphate, or any other calcium salt, as a byproduct from the reaction of tetracalcium phosphate, phosphoserine, and poly(lactide-co-glycolide) fibers in an aqueous medium. In some embodiments, the solidified form of the adhesive composition comprises hydroxyapatite, tetracalcium phosphate, tricalcium phosphate, or any other calcium salt, as a byproduct from the reaction of tetracalcium phosphate, phosphoserine, and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate powder, phosphoserine powder, poly(lactide-co-glycolide), and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, and a biologically active substance in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, poly(lactide-co-glycolide), and a biologically active substance in an aqueous medium.

In some embodiments, the additive is a polymer. Suitable polymers incorporated as additives into the adhesive composition may contain a functional group comprising an electronegative atom, e.g., as the bonding site of the polymer surface to the available metal ion, such as a carbonyl oxygen atom (e.g., of an ester) or a nitrogen atom (e.g., of an amine). These functional groups can be either in the backbone chain of the polymer or in groups pendant to the polymer chain. These polymeric based compounds may include, but are not limited to, a poly(L-lactide), poly(D,L-lactide), polyglycolide, poly(ε-caprolactone), poly(teramethylglycolic-acid), poly(dioxanone), poly(hydroxybutyrate), poly(hydroxyvalerate), poly(lactide-co-glycolide), poly(glycolide-co-trimethylene carbonate), poly(glycolide-co-caprolactone), poly(glycolide-co-dioxanone-co-trimethylene-carbonate), poly(tetramethylglycolic-acid-co-dioxanone-co-trimethylenecarbonate), poly(glycolide-co-caprolactone-co-lactide-co-trimethylene-carbonate), poly(hydroxybutyrate-co-hydroxyvalerate), poly(methylmethacrylate), poly(acrylate), a polyamine, a polyamide, a polyimidazole, poly(vinyl-pyrrolidone), collagen, silk, chitosan, hyaluronic acid, collagen, gelatin and/or mixtures thereof. In addition, co-polymers of the above homopolymers also can be used.

The general structural nature of a polymer (*e.g*., a polymer used as an additive in an adhesive composition described herein) may include a linear homopolymer and copolymer, a cross linked polymer, a block polymer, a branched polymer, a hyper branched polymer, or a star shaped polymer. The polymers can be added to the formulation in the form of a solution, powder, fiber, resin, liquid crystal, hydrogel, chip, flake, and the like. The polymer can be included directly within the adhesive composition or can be an adjunct that is applied in situ as the adhesive composition is applied per its method of use (*e.g.,* to attach to bone).

In some embodiments, certain additives may be provided as powders or granules or solutes or any combination thereof. These powders may exhibit a mean particle size of about 0.001 to about 0.750 mm, about 0.005 to about 0.150 mm, about 0.250 to about 0.750 mm, 0.250 to about 0.500, 0.015 to about 0.050 mm, about 0.015 to about 0.025 mm, about 0.020 to about 0.060 mm, about 0.020 to about 0.040 mm, about 0.040 to about 0.100 mm, about 0.040 to about 0.060 mm, about 0.060 to about 0.150 mm, or about 0.060 to about 0.125 mm. The mean particle size may be bi-modal to include any combination of mean particle sizes as previously described. These granules may exhibit a mean granule size of about 0.050 mm to about 5 mm, about 0.100 to about 1.500 mm, about 0.125 to 1.000 mm, 0.125 to 0.500 mm, about 0.125 to 0.250 mm, about 0.250 to 0.750 mm, about 0.250 to 0.500 mm, about 0.500 to 1.00 mm, about 0.500 to 0.750 mm. The mean granule size may be multi-modal to include any combination of mean granule sizes as previously described. In some embodiments, varying sizes of said powders or granules may be used in the adhesive composition.

In some embodiments, certain additives are provided as fibers. In some embodiments, the fibers exhibit a mean fiber diameter of about 0.010 mm to about 2 mm, about 0.010 mm to about 0.50 mm, or about 0.025 mm to about 0.075 mm. These fibers may exhibit a mean fiber length of about 0.025 mm to about 50.0 mm, about 0.50 mm to 10 mm, or about 1.00 mm to about 3.50 mm. The mean fiber diameter or length may be multi-modal to include any combination of mean fiber diameter or length as previously described.

In some embodiments, certain additives supplied as granules or fibers are porous. In some embodiments, certain additives supplied as granules or fibers are non-porous. The porous additive may have porosity that is interconnected or closed. The size of the pore in the porous additive may range in size from the nanometer range (e.g., about 10 nm to about 1000 nm) to micrometer range (e.g., about 10 µm to about 1000 µm) to the millimeter range (e.g., about 1 mm to about 10 mm). The total porosity of the additive may range from about 5% porosity to about 95% porosity. In some embodiments, the porosity of the additive is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% porosity.

In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, and poly(lactide-co-glycolide) in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, poly(lactide-co-glycolide), and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, and a biologically active substance in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, poly(lactide-co-glycolide), and a biologically active substance in an aqueous medium.

In some embodiments, the disease or condition comprises a cancer (*e.g.,* osteosarcoma), osteoporosis, rickets, osteogenesis imperfecta, fibrous dysplasia, Paget's disease of the bone, hearing loss, renal osteodystrophy, a malignancy of the bone, infection of the bone, severe and handicapping malocclusion, osteonecrosis, cleft palate, or other genetic or developmental disease. In some embodiments, the method comprises repair of a defect in a bone caused by a disease or condition, such as cancer (e.g., osteosarcoma), osteoporosis, rickets, osteogenesis imperfecta, fibrous dysplasia, Paget's disease of the bone, hearing loss, renal osteodystrophy, a malignancy of the bone, infection of the bone, or other genetic or developmental disease. In some embodiments, the method comprises strengthening a bone in a subject that has been weakened by a disease or condition, such as cancer (e.g., osteosarcoma), osteoporosis, rickets, osteogenesis imperfecta, Paget's disease of the bone, hearing loss, renal osteodystrophy, a malignancy of the bone, infection of the bone, or other genetic or developmental disease. In some embodiments, the subject has experienced a trauma, such as a broken bone, fractured bone, or damaged tooth. In some embodiments, the subject has experienced tooth decay. In some embodiments, the subject has experienced back, leg, arm, or neck pain, e.g., and is indicated for spinal fusion.

Fabricating the device in the form of a patch, plug, beam, plate, screw, rod, granule, spacer, cage, disc, tape, or other shape determined by the geometry or anatomy of the site of application, may comprise the following steps:
(1) dissolving or suspending a three dimensional fiber network material in a solvent resulting in formation of a solution;
(2) suspending the components of an adhesive composition in the solution or suspension prepared in step (1);
(3) suspending other solid particles into the suspension formed in step (2);
(4) mixing the suspension formed in step (3);
(5) fully or partially filling or casting the suspension formed in step (4) into a mold or container that defines the outer shape of a device;
(6) removing (e.g., selectively removing) the solvent of the suspension of step (5) through evaporation, which may be enhanced by partial vacuum or application of heat to recover or reconstitute the solid device comprised of the components of the adhesive composition of step (2) and the other solid particles of step (3) interspersed in a matrix of the three dimension fiber network material; and/or
(7) removing (e.g., selectively removing) the other solid particles of step (3) to produce a porous adhesive device.

Fabricating the device in the form of a patch, plug, beam, plate, screw, rod, granule, spacer, cage, disc, tape device, or other shape determined by the geometry or anatomy of the site of application, wherein the device comprises a solidified form of the adhesive composition, may comprise the following steps:
(1) preparing a mixture of powders (e.g., adhesive composition powders) and optionally adding an additive;
(2) adding an aqueous medium to the mixture of powders from step (1) to form an adhesive composition;
(3) fully or partially filling or casting the adhesive composition of step (3) into a mold or container or onto an outer surface that defines the outer shape of a device;
(4) allowing the adhesive composition to solidify;
(5) reshaping (e.g., selectively reshaping) the device to incorporate a geometric feature (e.g. a hole, threads, or tunnel) into or onto the device, and/or milling the device to a desired powder or granule size; and
(6) impregnating or coating the device with an adhesive composition.

In some embodiments, the device is prepared through a three-dimensional printing method (e.g., binder jetting).

### BRIEF DESCRIPTION OF THE DRAWINGS

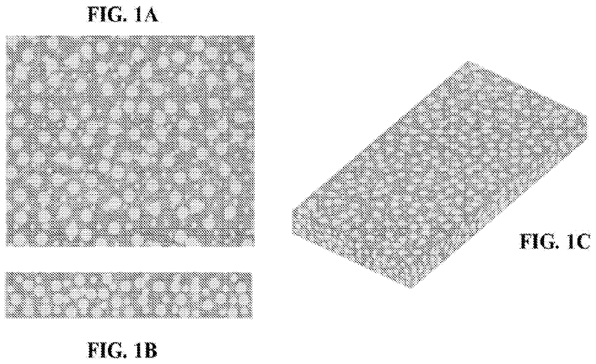

**FIGS. 1A-1C** are illustrations of the top (FIG. 1A), side (FIG. 1B), and isometric (FIG. 1C) views of an exemplary, but not claimed, three dimensional fiber network material comprised of fibers (random orientation) and an impregnated exemplary adhesive composition (in gray).

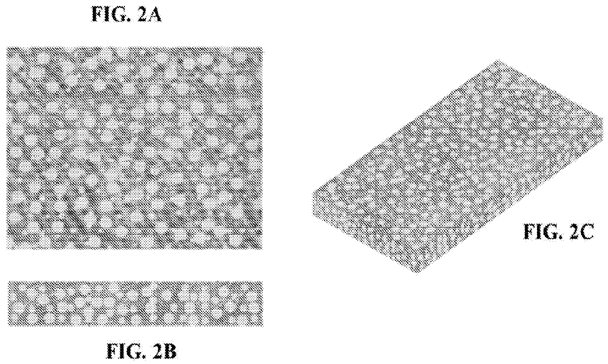

**FIGS. 2A-2C** are illustrations of the top (FIG. 2A), side (FIG. 2B), and isometric (FIG. 2C) views of an exemplary, but not claimed, three dimensional fiber network material comprised of fibers (both random and parallel orientations) and an impregnated exemplary adhesive composition (in gray).

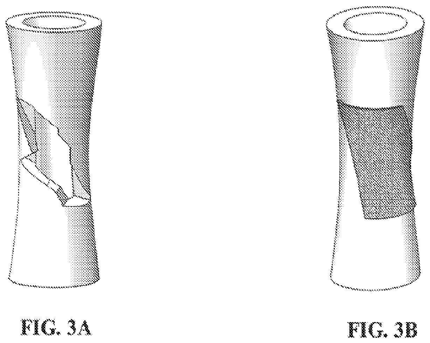

**FIGS. 3A and 3B** show isometric views of a surface spanning defect in an object (FIG. 3A) and the use of an exemplary, but not claimed, device comprising a three dimensional fiber network material impregnated with an adhesive composition (in gray) applied along the defect, wherein the device is operating as a patch (FIG. 3B).

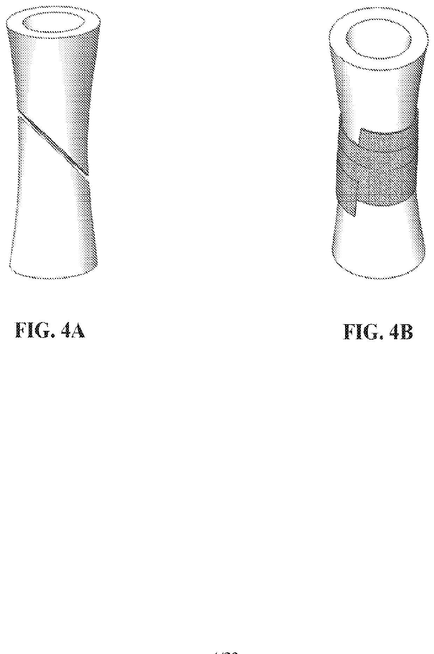

**FIGS. 4A and 4B** show isometric views of a surface spanning space between two objects (FIG. 4A) and the use of an exemplary, but not claimed, device comprising a three dimensional fiber network material impregnated with an adhesive composition (in gray) wrapped around region of discontinuity to provide fixation, wherein the device is operating as a tape (FIG. 4B).

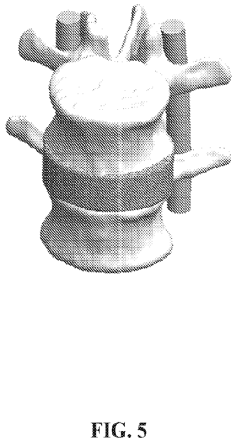

**FIG. 5** shows an isometric view of an exemplary device comprising a three dimensional fiber network material impregnated with an exemplary adhesive composition (in gray) applied as an interposition device between two objects, wherein the device is operating as a patch or plug.

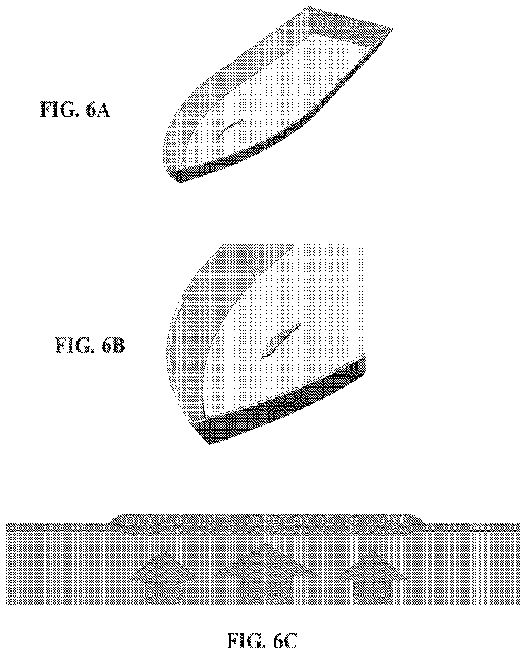

**FIGS. 6A-6C** show a defect in an object (FIG. 6A) and the use of an exemplary device comprising a three dimensional fiber network material impregnated with an exemplary adhesive composition (in gray) applied to the exterior surface of the defect (FIG. 6B, isometric view; FIG. 6C, side view), wherein the device is operating as a plug.

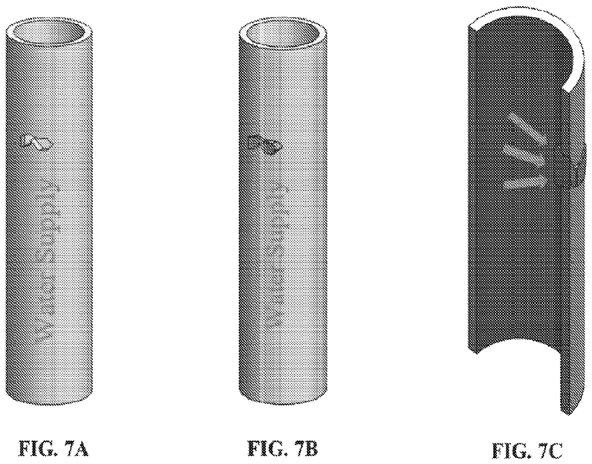

**FIGS. 7A-7C** show a defect in an object (FIG. 7A) and the use of an exemplary device comprising a three dimensional fiber network material impregnated with an exemplary adhesive composition (in gray) applied to the interior surface of the defect (FIG. 7B, exterior view; FIG. 7C, interior view), wherein the device is operating as a patch or plug.

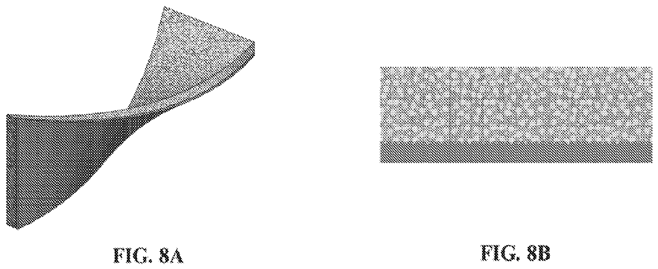

**FIGS. 8A and 8B** show the isometric (FIG. 8A) and side (FIG. 8B) views of an exemplary layered device comprising a three dimensional fiber network material impregnated with an exemplary adhesive composition (in gray) attached to an impermeable membrane, wherein the two layers are connected through mechanical interlocking on one side of the fiber network material.

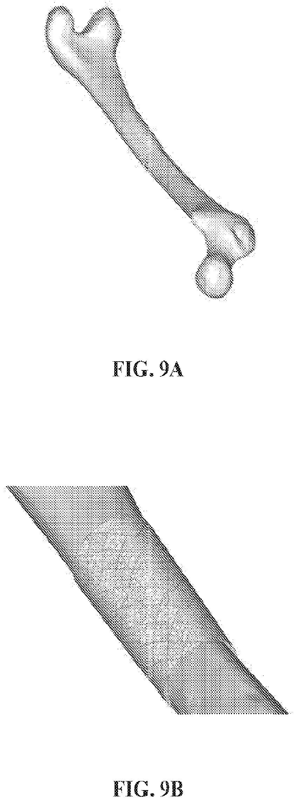

**FIGS. 9A and 9B** show the overview (FIG. 9A) and close up (FIG. 9B) views of an exemplary but not claimed device impregnated with an exemplary adhesive composition in the form of a cylindrical cuff joining two segments of a fractured bone.

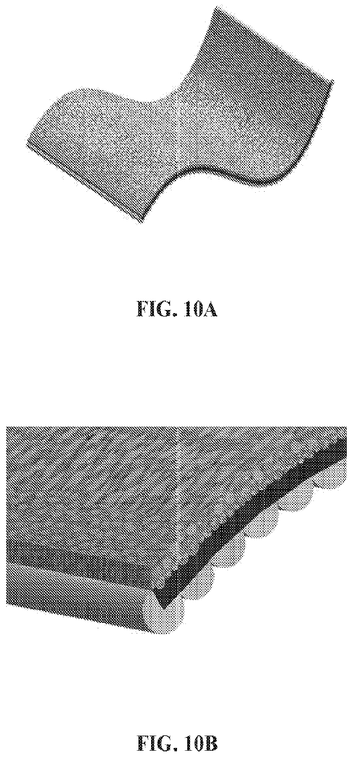

**FIGS. 10A and 10B** show the isometric (FIG. 10A) and side (FIG. 10B) views of an exemplary device comprising a mesh component (in dark gray, middle layer) and impregnated with an exemplary adhesive composition (in light gray) that is substantially rigid in one dimension and substantially compliant in another (e.g., an orthogonal) dimension.

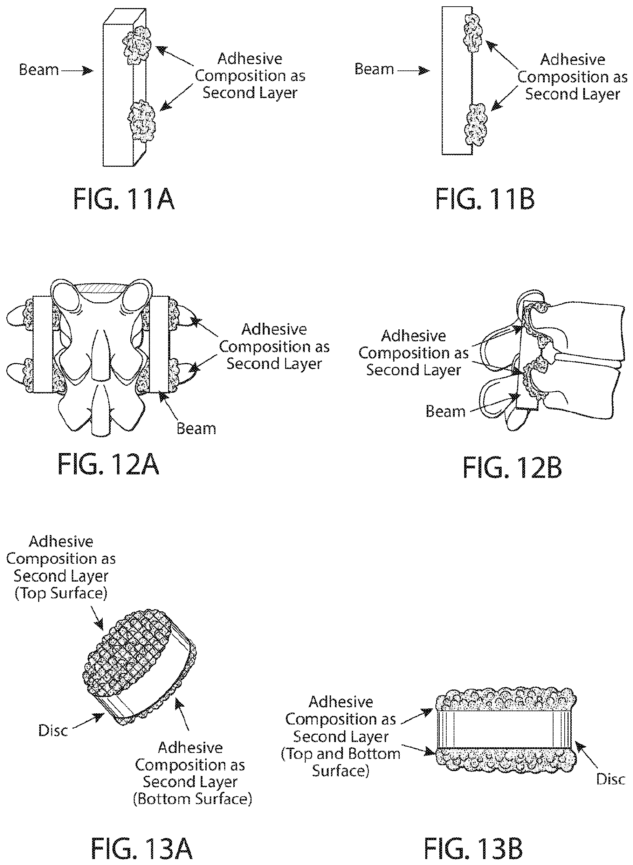

**FIGS. 11A and 11B** show the isometric (FIG. 11A) and side (FIG. 11B) views of an exemplary device comprising a solidified beam made of an exemplary adhesive composition with a second layer of adhesive composition applied as a coating to the surface of the beam in the working state, e.g., to provide a means of primary fixation of the beam upon curing.
**FIG. 12A and 12B** show the posterior (FIG. 12A) and lateral (FIG. 12B) views of posterolateral lumbar fixation of adjacent transverse processes with application of an exemplary device. The device comprises a second layer of an exemplary adhesive composition applied as a coating to the surface of the solidified beam in the working state. The second layer the exemplary adhesive composition provides primary fixation of the adhesive device to both the adjacent transverse processes and along the *pars interarticularis* upon curing.
**FIGS. 13A and 13B** show the isometric (FIG. 13A) and side (FIG. 13B) views of an exemplary device comprising a solidified disc of the adhesive composition and a second layer of adhesive composition applied as a coating to the top and bottom surfaces of the disc in the working state, e.g., to provide a means of primary fixation of the disc upon curing.

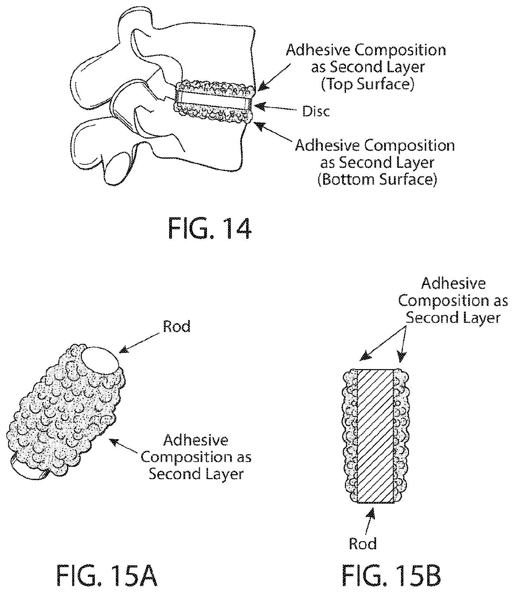

**FIG. 14** shows the side view of interbody fixation of adjacent spinal vertebral bodies with application of an exemplary device. The adhesive device comprises a second layer of an exemplary adhesive composition and applied as a coating to the top and bottom surfaces of the solidified disc in the working state. The second layer provides primary fixation of the adhesive device to the adjacent vertebral bodies upon curing.
**FIGS. 15A-15B** show the isometric (FIG. 15A) and side (FIG. 15B) views of an exemplary device comprising a solidified rod of an exemplary adhesive composition and a second layer of an adhesive composition applied as a coating to the surface of the rod in the working state.

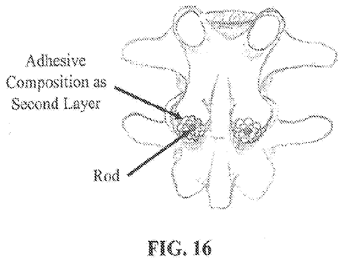

**FIG 16** shows the side view of facet joint fixation of adjacent spinal vertebral bodies containing an exemplary device. The device is held in place through a layer of an exemplary adhesive composition that was added prior to placement of the device to adjacent vertebral bodies.

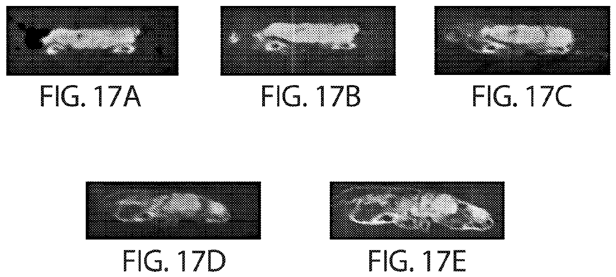

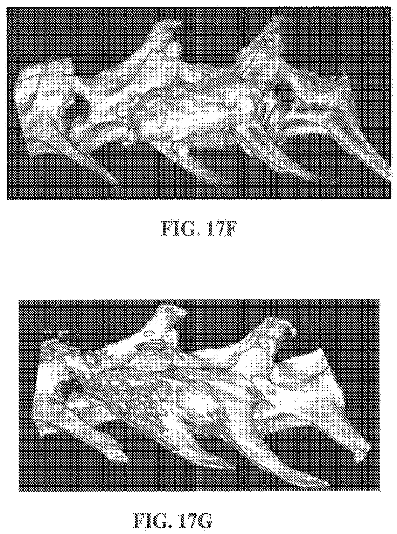

**FIGS. 17A-17G** are a series of CBCT reconstructed images from a study of posterolateral lumbar spinal fusion whereby adjacent rabbit spinal vertebral bodies (L5/L6) were fixed bilaterally with Device A from Example 2. The following time points show parasagittal plane images through Device A, immediately post-surgical (FIG. 17A), 6 weeks (FIG. 17B), 16 weeks (FIG. 17C), 31 weeks (FIG. 17D) and 39 weeks (FIG. 17D). Dorsolateral view of three-dimensional reconstructions of the CBCT data at immediately post-surgical intervention and at 39 weeks of post-surgical intervention (FIG. 17F-17G).

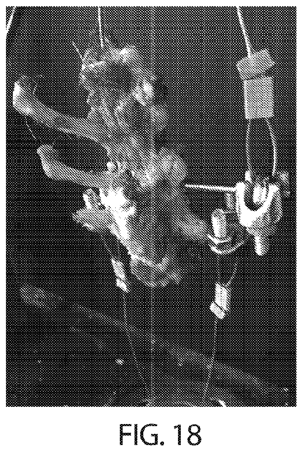

**FIG. 18** is an image of an explanted spinal tissue segment at the 10-week post-implantation time point mounted on an Instron machine for tensile testing, whereby adjacent rabbit spinal vertebral bodies (L5/L6) were fixed bilaterally with Device A from Example 2 to bridge the gap between transverse processes.

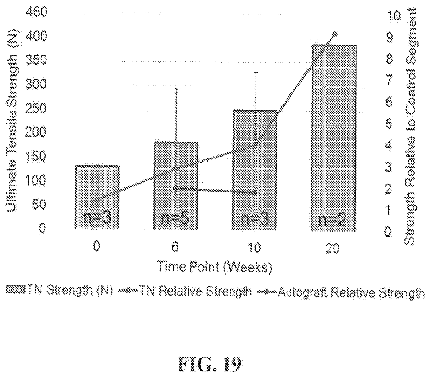

**FIG. 19** shows mechanical tensile test results from a 20-week *in vivo* study of posterolateral lumbar spinal fusion in which adjacent rabbit spinal vertebral bodies (L5/L6) were fixated bilaterally with Device A from Example 2 and explanted for mechanical tensile testing at various time points post-implantation.

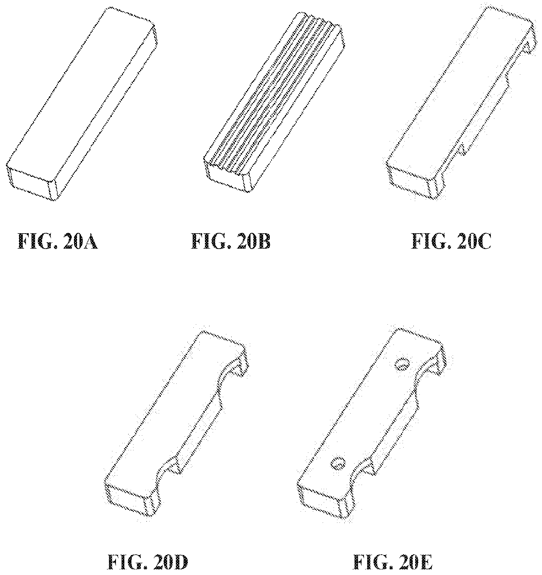

**FIGS. 20A-20E** show a series of isometric representations of an exemplary device, a beam (FIG. 20A), with optional modifications, rib features (FIG. 20B), reliefs for anatomical features on inferior surface (FIG. 20C), reliefs for anatomical features on both the inferior and lateral surfaces (FIG. 20D), and reliefs for anatomical features on both the inferior and lateral surfaces and injection ports for adhesive (FIG. 20E).

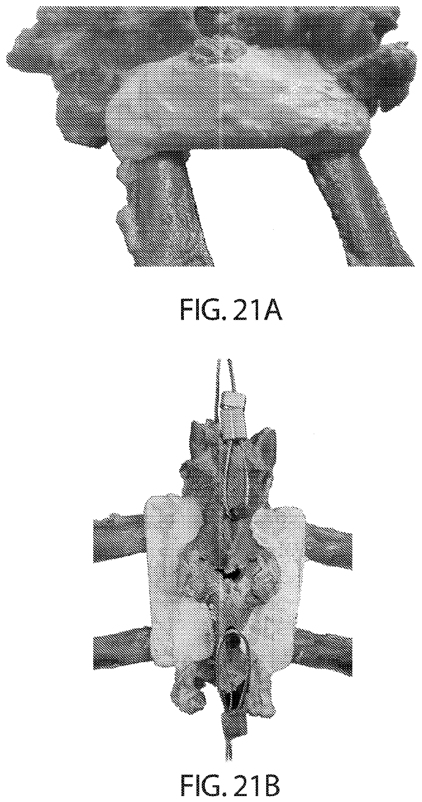

**FIGS. 21A-B** show lateral (FIG. 21A) and dorsal (FIG. 21B) views of Device E, as described in Example 5, being used in in spinal fixation (inter-transverse process and inter articular process) in cadaveric sheep.

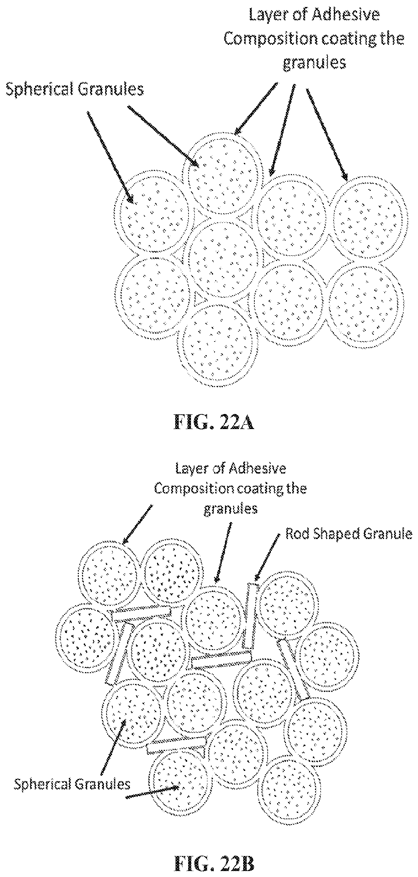

**FIGS. 22A-22B** show two-dimensional schematic views illustrating the packing of substantially uniform spherical granules with an additional layer of adhesive composition coating the granules (FIG. 22A) and a combination of spherical and rod-shaped granules with an additional layer of adhesive composition coating the granules and rods (FIG. 22B). Note the interstitial spaces between the granules.

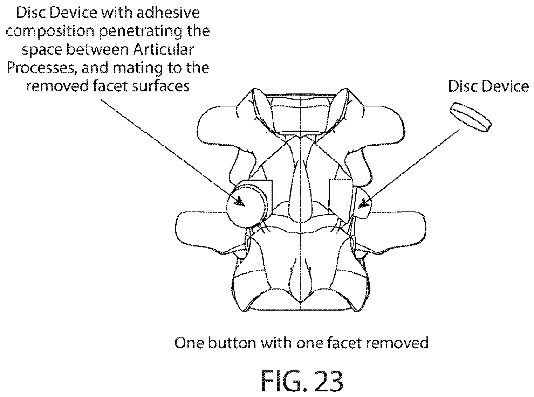

**FIG. 23** shows a dorsal view of the spine demonstrating spinal fixation between the articular processes using Device F, as described in Example 6.

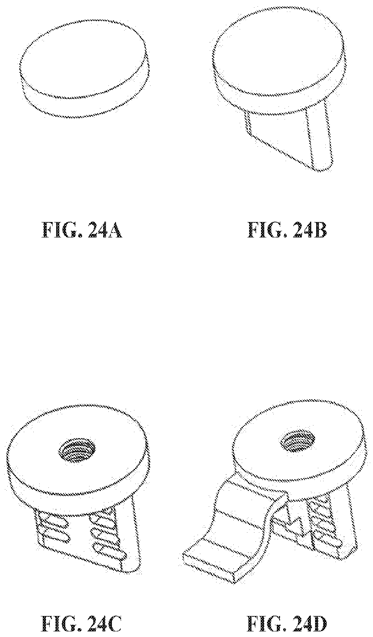

**FIGS. 24A-D** show a series of isometric representations of an exemplary device, a disc (FIG. 24A), with optional modifications, stabilizer extension (FIG. 24B), stabilizer extension with perforations and an injection port (FIG. 24C), primary stabilizer extension with perforations, secondary stabilizer extension, and an injection port (FIG. 24D).

### DETAILED DESCRIPTION

### Components of Adhesive Devices

The device may be in the form of a patch, plug, beam, plate, screw, rod, granule, spacer, cage, disc, tape device, or other shape determined by the geometry or anatomy of the site of application. The device comprises a solidified form of an adhesive composition after the reaction has completed in the presence of an aqueous medium, and a three dimensional fiber network. The device comprises an additional layer or layers of the adhesive composition in its working state (i.e., after addition of aqueous medium becomes tacky and adhesive) that is coated or impregnated into or onto the surface of the solidified form and allowed to cure during its method of use.

The method defined herein is for joining separated bones. The method may be for treating or heal a subject suffering from a disease or condition.

### A. Three dimensional fiber network materials

The three dimensional fiber network material for use in the disclosed invention may take several forms, including, but not limited to a two or three-dimensional grid, lattice, mesh, mat, weave, braid, cloth, fabric, felt, web, open cell foam, sponge, sheet, membrane, cage, or gel. Exemplary interconnected fiber networks are shown in FIGS. 1A-1C and 2A-2C.

In some embodiments, the three dimensional fiber network material comprises elements, threads, or components that are structurally interconnected or intertwined (e.g., braided or woven together). For example, the elements, threads, or components of the three dimensional fiber network material may be interconnected through mechanical entanglement, chemical bonding (e.g., covalent bonding, metallic bonding, ionic bonding, ionomeric bonding, coordination bonding), or intertwined to form larger bundles of fibers. In some embodiments, the point of interconnection or intertwinement of the elements is referred to herein as a node, and the distance between two or more nodes is referred to herein as the node length.

The three dimensional fiber network material may comprise elements of varied composition, each of which may comprise a different shape or dimension (e.g., diameter, length, aspect ratio). For example, in some embodiments, the mean diameter of the fiber network element is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the mean diameter of the fiber network element is from about 10 to about 500 nanometers, from about 25 to about 500 nanometers, from about 50 to about 500 nanometers, or from about 100 to about 500 nanometers.

In other embodiments, the mean diameter of the fiber network element is in the micrometer range, e.g., from 1 to 1000 micrometers, from 1 to about 750 micrometers, from 1 to 500 micrometers, from 1 to about 250 micrometers, from 1 to 100 micrometers, or from 1 to about 50 micrometers. In some embodiments, the mean diameter of the fiber network element is from about 10 to 500 micrometers, from about 25 to 500 micrometers, from about 50 to 500 micrometers, or from 100 to 500 micrometers.

In still other embodiments, the mean diameter of the fiber network element is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the mean diameter of the fiber network element is from about 1 to about 10 millimeters, from about 1 to about 7.5 millimeters, from about 1 to about 5 millimeters, or from about 1 to about 2.5 millimeters.

The three dimensional fiber network material may comprise nodes of various node lengths. For example, in some embodiments, the node length of the fiber network is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the node length of the fiber network is from about 10 to about 500 nanometers, from about 25 to about 500 nanometers, from about 50 to about 500 nanometers, or from about 100 to about 500 nanometers.

In other embodiments, the node length of the fiber network is in the micrometer range, e.g., from about 1 to about 1000 micrometers, from about 1 to about 750 micrometers, from about 1 to about 500 micrometers, from about 1 to about 250 micrometers, from about 1 to about 100 micrometers, or from about 1 to about 50 micrometers. In some embodiments, the node length of the fiber network is from about 10 to about 500 micrometers, from about 25 to about 500 micrometers, from about 50 to about 500 micrometers, or from about 100 to about 500 micrometers.

In still other embodiments, the node length of the fiber network is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the node length of the fiber network is from about 1 to about 10 millimeters, from about 1 to about 7.5 millimeters, from about 1 to about 5 millimeters, or from about 1 to about 2.5 millimeters.

In some embodiments, the three dimensional fiber network material may comprise a small molecule component that ranges in size from about 10 Da to about 2,000 Da. For example, in some embodiments, the small molecule may range in size from about 25 Da to about 1750 Da, from about 50 Da to about 1500 Da, from about 100 Da to about 1250 Da, from about 100 Da to about 1000 Da, from about 100 to about 750 Da, or from about 100 Da to about 500 Da. In other embodiments, the three dimensional fiber network material may comprise a large molecule or polymer component that ranges in size from about 2,000 Da to about 500,000 Da. For example, in some embodiments, the large molecule or polymer may range in size from about 2,000 to about 400,000 Da, from about 2,000 Da to about 300,000 Da, from about 2,000 Da to about 200,000 Da, from about 2,000 Da to about 100,000 Da, from about 2,000 Da to about 75,000 Da, from about 2,000 Da to about 50,000 Da, from about 2,000 to about 25,000 Da, or from about 2,000 Da to about 10,000, or from about 2,000 Da to about 5,000. In some embodiments, the size of the small molecule, large molecule, or polymer component is selected to obtain a desirable property, e.g., increased strength or decreased bioresorption time.

The three dimensional fiber network material may comprise a substantially biocompatible or substantially bioresorbable component. Exemplary biocompatible or bioresorbable components may include, but are not limited to, calcium-based components (e.g., calcium phosphates, calcium sulfates), glass-ceramic or bioactive glass components (e.g., comprising SiO₂, Na₂O, CaO, or P₂O₅), or certain biopolymers (e.g., glycolic acid polyesters, lactic acid polyesters, copolymers of glycolic and lactic acid, collagen, gelatin, fibrin, polysaccharides (e.g., starch, amylose, amylopectin, dextran, glycogen, chitin, cellulose, polymers comprising glucosamine, or mucopolysaccharides (e.g., glycosaminoglycans, e.g., chondroitin)). In some embodiments, the biopolymer comprises poly(L-lactide), poly(D,L-lactide), poly(glycolide), poly(ε-caprolactone), poly(carbonate), poly(urethane), poly(ethylene), poly(teramethylglycolic-acid), poly(dioxanone), poly(hydroxybutyrate), poly(hydroxyvalerate), poly(L-lactide-co-glycolide), poly(glycolide-co-trimethylene-carbonate), poly(glycolide-co-caprolactone), poly(glycolide-co-dioxanone-co-trimethylene-carbonate), poly(tetramethylglycolic-acid-co-dioxanone-co-trimethylene-carbonate), poly(glycolide-co-caprolactone-co-L-lactide-co-trimethylene-carbonate), poly(hydroxybutyrate-co-hydroxyvalerate), poly(methyl-methacrylate), a polyamine, a polyimidazole, poly(vinyl-pyrrolidone), chitosan, hyaluronic acid, collagen, gelatin, or a copolymer, derivative, or mixture thereof.

In other embodiments, the three dimensional fiber network material comprises a substantially non-resorbable component. Exemplary non-resorbable components may include, but are not limited to, certain polymers or materials, e.g., silk, nylon, a polyamide, glass, carbon, an aromatic (e.g., polyphenylene vinylene) and/or conjugated (e.g., polyacetylene) polymer, an intrinsically conductive polymer (e.g., polyaniline, polypyrrole, polythiophene), a metal (e.g., calcium, silicon, copper, silver, gold, zinc, iron, titanium, aluminum, cobalt, chromium, tantalum, molybdenum), a metallic alloy (e.g., bronze, brass, steel (e.g., stainless steel), cobalt-chromium), poly(ether ketone), poly(ethylene), poly(urethane), poly(methyl methacrylate), or poly(acrylic acid) polymers or a copolymer, derivative, or mixture thereof.

In some embodiments, the three dimensional fiber network material may comprise a linear homopolymer, a linear copolymer, a cross linked polymer, a block polymer, a branched polymer, a hyper branched polymer, a star shaped polymer or a mixture thereof.

The three dimensional fiber network material may comprise an element that affects its physical or mechanical properties, e.g., the mechanical compliance, strength, or stiffness of the material. For example, in some embodiments, the three dimensional fiber network material comprise elements that provide increased mechanical compliance and is substantially compliant or deformable, e.g., elastic, plastic, or is able to conform to the shape, contour, and size of the objects to which it is applied. In some embodiments, the three dimensional fiber network material comprises elements that provide increased strength or stiffness, e.g., fibers, rods, or plates. In other embodiments, the three dimensional fiber network material comprises a self-adhesive element, e.g., wherein the element comprises loops and locks (e.g. Velcro^{®}). The elements within the three dimensional fiber network may be straight, curved, curled, looped, bent or any combination thereof. In one embodiment, the three dimensional fiber network material comprises a self-adhesive element, e.g., wherein the element comprises loops and locks (e.g. Velcro^{®}) and does not comprise an adhesive composition (e.g., an adhesive composition described herein).

In some embodiments, the three dimensional fiber network is substantially crosslinked, e.g., about 100%, about 90%, about 80%, about 70%, about 60%, about 50%, about 40%, about 30%, about 20%, about 10%, about 5%, or about 1% crosslinked. In other embodiments, the three dimensional fiber network material is substantially non-crosslinked, e.g., about 100%, about 90%, about 80%, about 70%, about 60%, about 50%, about 40%, about 30%, about 20%, about 10%, about 5%, or about 1% non-crosslinked. In some embodiments, the three dimensional fiber network material is able to withstand or resist a force (e.g., direct impact force, static force, or dynamic force) or stress of at least about 6.9 kPa (1 psi), 34 kPa (5 psi), about 69 kPa (10 psi), about 100 kPa (15 psi), about 140 kPa (20 psi), about 170 kPa (25 psi), about 340 kPa (50 psi) or more, depending upon the application for which it is intended. In some embodiments, the three dimensional fiber network material is able to withstand or resist a pressure (e.g., a hydrostatic pressure) of at least about 6.9 kPa (1 psi), 34 kPa (5 psi), about 69 kPa (10 psi), about 100 kPa (15 psi), about 140 kPa (20 psi), about170 kPa ( 25 psi), about 340 kPa (50 psi) or more, depending upon the application for which it is intended.

The three dimensional fiber network material may comprise a mesh component, e.g., a membrane, sheet, coating, fiber, rod, plate, or strut. In some embodiments, the three dimensional fiber network material may comprise a plurality of mesh components, e.g., membranes, sheets, coatings, fibers, rods, plates, or struts. The mesh components may be permeable and allow for movement of particulates, fluids, gases, or other substances, or the mesh components may be substantially impermeable and prevent movement of particulates, fluids, gases, or other substances, relative to other mesh components. The mesh component may be situated on one or more sides or faces of the three dimensional fiber network material. In other embodiments, the mesh component may be embedded within or throughout the interconnected fiber network. In some embodiments, the mesh component is substantially compliant or deformable, e.g., elastic, plastic, or able to conform to the shape and sizes of the objects to which it is applied. In other embodiments, the mesh component is substantially rigid or stiff. In still other embodiments, the mesh component is substantially rigid or stiff prior to the application of the device, but then adopts a substantially compliant or deformable state upon application. In some embodiments, the mesh component is substantially rigid in one dimension while it is substantially compliant in another (e.g., an orthogonal) dimension (e.g., as shown in FIGS. 10A and 10B).

The mesh component may comprise elements of varied composition, each of which may comprise a different shape or dimension (e.g., diameter, node length). For example, in some embodiments, the mean diameter of the mesh component element is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the mean diameter of the mesh component element is from about 10 to about 500 nanometers, from about 25 to about 500 nanometers, from about 50 to about 500 nanometers, or from about 100 to about 500 nanometers.

In other embodiments, the mean diameter of the mesh component element is in the micrometer range, e.g., from about 1 to about 1000 micrometers, from about 1 to about 750 micrometers, from about 1 to about 500 micrometers, from about 1 to about 250 micrometers, from about 1 to about 100 micrometers, or from about 1 to about 50 micrometers. In some embodiments, the mean diameter of the mesh component element is from about 10 to about 500 micrometers, from about 25 to about 500 micrometers, from about 50 to about 500 micrometers, or from about 100 to about 500 micrometers.

In still other embodiments, the mean diameter of the mesh component element is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the mean diameter of the mesh component element is from about 1 to about 10 millimeters, from about 1 to about 7.5 millimeters, from about 1 to about 5 millimeters, or from about 1 to about 2.5 millimeters.

The mesh component material may comprise nodes of various node lengths. For example, in some embodiments, the node length of the mesh component is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the node length of the mesh component is from about 10 to about 500 nanometers, from about 25 to about 500 nanometers, from about 50 to about 500 nanometers, or from about 100 to about 500 nanometers.

In other embodiments, the node length of the mesh component is in the micrometer range, e.g., from about 1 to about 1000 micrometers, from about 1 to about 750 micrometers, from about 1 to about 500 micrometers, from about 1 to about 250 micrometers, from about 1 to about 100 micrometers, or from about 1 to about 50 micrometers. In some embodiments, the node length of the mesh component is from about 10 to about 500 micrometers, from about 25 to about 500 micrometers, from about 50 to about 500 micrometers, or from about 100 to about 500 micrometers.

In still other embodiments, the node length of the mesh component is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the node length of the mesh component is from about 1 to about 10 millimeters, from about 1 to about 7.5 millimeters, from about 1 to about 5 millimeters, or from about 1 to about 2.5 millimeters.

In some embodiments, the mesh component comprises a small molecule component that ranges in size from about 10 Da to about 2,000 Da. For example, in some embodiments, the small molecule ranges in size from about 25 Da to about 1750 Da, from about 50 Da to about 1500 Da, from about 100 Da to about 1250 Da, from about 100 Da to about 1000 Da, from about 100 to about 750 Da, or from about 100 Da to about 500 Da. In other embodiments, the mesh component comprises a large molecule or polymer component that ranges in size from about 2,000 Da to about 500,000 Da. For example, in some embodiments, the large molecule or polymer ranges in size from about 2,000 to about 400,000 Da, from about 2,000 Da to about 300,000 Da, from about 2,000 Da to about 200,000 Da, from about 2,000 Da to about 100,000 Da, from about 2,000 Da to about 75,000 Da, from about 2,000 Da to about 50,000 Da, from about 2,000 to about 25,000 Da, or from about 2,000 Da to about 10,000, or from about 2,000 Da to about 5,000. In some embodiments, the size of the small molecule, large molecule, or polymer component is selected to obtain a desirable property, e.g., increased strength or decreased biodegradation or resorption time.

The mesh component may comprise a substantially biocompatible or substantially bioresorbable component. Exemplary biocompatible or bioresorbable components may include, but are not limited to, calcium-based components (e.g., calcium phosphates, calcium sulfates), glass-ceramic or bioactive glass components (e.g., comprising SiO₂, Na₂O, CaO, or P₂O₅), or certain biopolymers (e.g., glycolic acid polyesters, lactic acid polyesters, copolymers of glycolic and lactic acid, collagen, fibrin, polysaccharides (e.g., starch, amylose, amylopectin, glycogen, dextran, chitin, cellulose, polymers comprising glucosamine, or mucopolysaccharides (e.g., glycosaminoglycans, e.g., or chondroitin)). In some embodiments, the biopolymer comprises poly(L-lactide), poly(D,L-lactide), poly(glycolide), poly(ε-caprolactone), poly(carbonate), poly(urethane), poly(ethylene), poly(teramethylglycolic-acid), poly(dioxanone), poly(hydroxybutyrate), poly(hydroxyvalerate), poly(L-lactide-co-glycolide), poly(glycolide-co-trimethylene-carbonate), poly(glycolide-co-caprolactone), poly(glycolide-co-dioxanone-co-trimethylene-carbonate), poly(tetramethylglycolic-acid-co-dioxanone-co-trimethylene-carbonate), poly(glycolide-co-caprolactone-co-L-lactide-co-trimethylene-carbonate), poly(hydroxybutyrate-co-hydroxyvalerate), poly(methyl-methacrylate), poly(acrylate), a polyamine, a polyimidazole, poly(vinyl-pyrrolidone), chitosan, hyaluronic acid, collagen, gelatin, or a copolymer, derivative, or mixture thereof.

In some embodiments, the mesh component comprises a substantially non-resorbable component. Exemplary non-resorbable or non-biocompatible components may include, but are not limited to, certain polymers or materials, e.g., silk, nylon, polyamides, glass, carbon, an aromatic (e.g., polyphenylene vinylene) and/or conjugated (e.g., polyacetylene) polymer, an intrinsically conductive polymer (e.g., polyaniline, polypyrrole, polythiophene), a metal (e.g., calcium, silicon, copper, silver, gold, zinc, iron, titanium, aluminum, cobalt, chromium, tantalum, molybdenum), metallic alloys (e.g., bronze, brass, steel (e.g., stainless steel), cobalt-chromium), poly(ether ketone), poly(ethylene), poly(urethane), poly(methyl methacrylate), or poly(acrylic acid) or a copolymer, derivative, or mixture thereof.

The mesh component may comprise an element that affects its physical or mechanical properties, e.g., the mechanical compliance, strength, or stiffness of the material. For example, in some embodiments, the mesh component comprises elements that provide increased strength or stiffness, e.g., fibers, rods, or struts. In other embodiments, the mesh component comprises a self -adhesive element, e.g., wherein the element comprises loops and locks (e.g. Velcro^{®}). The elements within the mesh component may be straight, curved, curled, looped, bent or any combination thereof. In one embodiment, the mesh component comprises a self-adhesive element, e.g., wherein the element comprises loops and locks (e.g. Velcro^{®}) and does not comprise an adhesive composition (e.g., an adhesive composition described herein).

In some embodiments, the mesh component is substantially entangled or crosslinked, e.g., about 100%, about 90%, about 80%, about 70%, about 60%, about 50%, about 40%, about 30%, about 20%, about 10%, about 5%, or about 1% entangled or crosslinked. In some embodiments, the mesh component is substantially non-entangled or non-crosslinked, e.g., about 100%, about 90%, about 80%, about 70%, about 60%, about 50%, about 40%, about 30%, about 20%, about 10%, about 5%, or about 1% non-entangled or non-crosslinked. In some embodiments, the mesh component is able to withstand or resist a pressure (e.g., a hydrostatic pressure) of at least about 6.9 kPa (1 psi), 34 kPa (5 psi), about 69 kPa (10 psi), about 100 kPa (15 psi), about 140 kPa (20 psi), about 170 kPa (25 psi), about 340 kPa (50 psi) or more, depending upon the application for which it is intended. In some embodiments, the mesh component is able to withstand or resist a force (e.g., a direct force, static force, or dynamic force) or a stress of at least about 6.9 kPa (1 psi), 34 kPa (5 psi), about 69 kPa (10 psi), about 100 kPa (15 psi), about 140 kPa (20 psi), about 170 kPa (25 psi), about 340 kPa (50 psi) or more, depending upon the application for which it is intended.

In some embodiments, the mesh component is mechanically or physically coupled (e.g., attached to (e.g., cross-linked to)) the three dimensional fiber network material, e.g., in a manner to withstand a pressure or force or resist degradation.

In some embodiments, the adhesive composition or components thereof are mixed into about 1%, about 2.5%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or more of the three dimensional fiber network material or the mesh component. In some embodiments, the adhesive composition or components thereof substantially immobilize the elements of the three dimensional fiber network material or the mesh component upon introduction of an aqueous medium or upon curing.

Exemplary three dimensional fiber network materials or mesh components that may be used in the adhesive device (e.g., as described herein) include, but are not limited to, gauze pads (e.g., NuGauze^{®}), field wound dressings, scouring pads, absorbable compressed sponges (e.g., Gel Foam^{®}), collagen matrices (e.g., Matrixflex^{™}, MatrixMem^{™}, MatrixDerm^{®}, MatrixDerm^{®} EXT, MatrixDerm^{®} Cap), polystyrene matrices (e.g., Styrofoam^{™}), bandages (e.g., elastic bandages (e.g., ACE^{™} bandages) or adhesive bandages (e.g., BAND-AID^{®} bandages)), cable ties (e.g., resorbable nylon cable ties), and adhesive tapes (e.g., masking tape, duct tape, electrical tape, or cellophane tape).

### B. Adhesive Compositions

The adhesive device of the present disclosure comprises a solidified form of an adhesive composition and a three dimensional fiber network. The device comprises an additional layer of the adhesive composition coated onto the device, or impregnated into or onto the surface of the device. This additional layer of the adhesive composition provides a means of adhering the device to another object, and solidifies during its method of use. The additional layer of the adhesive composition is present in the working state.

In some embodiments, the adhesive composition comprises a multivalent metal salt (e.g., one or more multivalent salts) and an acidic compound in an aqueous environment, e.g., an aqueous medium. In other embodiments, the adhesive composition comprises a multivalent metal salt (e.g., one or more multivalent salts) and an acidic compound in the absence of an aqueous environment, e.g., an aqueous medium. In the method defined herein, an aqueous medium is added to the adhesive composition or the components of the adhesive composition prior to the use of the device. In the presence of an aqueous environment, e.g., an aqueous medium, the adhesive composition reacts entering its working state (i.e., becomes tacky and adhesive) and cures to a solid state.

Multivalent metal salts including calcium phosphates (*e.g*., tetracalcium phosphate) have been shown to react with an acidic compound in aqueous environments, e.g., an aqueous medium, to form compositions with adhesive properties. Without wishing to be bound by theory, the multivalent metal salts are thought to form ionic interactions with the acidic compounds, which when combined in certain ratios react to provide a cement-like material. The multivalent metal salts and the acidic compounds may be locally blended and intermixed throughout the device in such a manner that allows the molecules of each component to dissolve and react with each other when subjected to an aqueous environment. Exemplary multivalent metal salts may be organic or inorganic in nature and include calcium phosphates (*e.g*., hydroxyapatite, octacalcium phosphate, tetracalcium phosphate, tricalcium phosphate), calcium nitrate, calcium citrate, calcium carbonate, magnesium phosphates, sodium silicates, lithium phosphates, titanium phosphates, strontium phosphates, barium phosphates, zinc phosphates, calcium oxide, calcium hydroxide, magnesium oxide, and combinations thereof.

The amount of each multivalent metal salt (*e.g.,* a calcium phosphate, calcium oxide, calcium hydroxide, or a combination thereof) may vary, *e.g*., between about 10% to about 90% weight by weight (w/w) of the total adhesive composition. In some embodiments, the amount of the multivalent metal salt (*e.g.,* a calcium phosphate, calcium oxide, calcium hydroxide, or a combination thereof) is in the range of about 10% to about 90%, about 15% to about 85%, about 20% to about 80%, about 30% to about 75%, about 40% to about 70%, or about 50% to about 65% w/w of the total adhesive composition. In other embodiments, the amount of the metal salt (*e.g.,* a calcium phosphate, calcium oxide, calcium hydroxide, or a combination thereof) is in the range of about 5% to about 95%, about 10% to about 85%, about 15% to about 75%, about 20% to about 65%, about 25% to about 55%, or about 35% to about 50% w/w of the total adhesive composition.

The adhesive composition comprises at least one multivalent metal salt (*e.g.,* a calcium phosphate, calcium oxide, calcium hydroxide, or a combination thereof). In other embodiments, the adhesive composition comprises at least two multivalent metal salts (*e.g.,* a calcium phosphate, calcium oxide, calcium hydroxide, or a combination thereof).

Acidic compounds of the adhesive composition are of Formula (I) or a salt thereof: 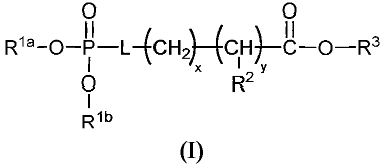 wherein L is O, S, NH, or CH₂; each of R^{1a} and R^{1b} is independently H, optionally substituted alkyl, or optionally substituted aryl; R² is H, NR^{4a}R^{4b}, C(O)R⁵, or C(O)OR⁵; R³ is H, optionally substituted alkyl, or optionally substituted aryl; each of R^{4a} and R^{4b} is independently H, C(O)R⁶, or optionally substituted alkyl; R⁵ is H, optionally substituted alkyl, or optionally substituted aryl; R⁶ is optionally substituted alkyl or optionally substituted aryl; and each of x and y is independently 0, 1, 2, or 3.

In some embodiments, L is O or S. In some embodiments, L is O. In some embodiments, each of R^{1a} and R^{1b} is independently H. In some embodiments, L is O, and each of R^{1a} and R^{1b} is independently H. In some embodiments, R² is H, NR^{4a}R^{4b}, or C(O)R⁵. In some embodiments, R² is NR^{4a}R^{4b}. In some embodiments, R² is NR^{4a}R^{4b} and each of R^{4a} and R^{4b} is independently H. In some embodiments, L is O, each of R^{1a} and R^{1b} is H, R² is NR^{4a}R^{4b}, and each of R^{4a} and R^{4b} is independently H. In some embodiments, R³ is H. In some embodiments, L is O, each of R^{1a} and R^{1b} is independently H, R² is NR^{4a}R^{4b}, each of R^{4a} and R^{4b} is independently H, and R³ is H. In some embodiments, each of x and y is 0 or 1. In some embodiments, each of x and y is 1. In some embodiments, L is O, each of R^{1a} and R^{1b} is H, R² is NR^{4a}R^{4b}, each of R^{4a} and R^{4b} is independently H, R³ is H, and each of x and y is 1. In some embodiments, the compound of Formula (I) is phosphoserine.

As used herein, the term "optionally substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds (e.g., alkyl, alkenyl, alkynyl, cycloalkyl, heterocycloalkyl, aryl, or heteroaryl, any of which may itself be further substituted), as well as halogen, carbonyl (*e.g*., aldehyde, ketone, ester, carboxyl, or formyl), thiocarbonyl (*e.g*., thioester, thiocarboxylate, or thioformate), amino, -N(R^{b})(RC^{c}), wherein each R^{d} and R^{c} is independently H or C₁-C₆ alkyl, cyano, nitro, -SO₂N(R^{b})(R^{c}), -SOR^{d}, and S(O)₂R^{d}, wherein each R^{b}, R^{c}, and R^{d} is independently H or C₁-C₆ alkyl. Illustrative substituents include, for example, those described herein above. The permissible substituents can be one or more and the same or different for appropriate organic compounds. This disclosure is not intended to be limited in any manner by the permissible substituents of organic compounds. It will be further understood that "substitution" or "substituted with" includes the implicit proviso that such substitution is in accordance with permitted valence of the substituted atom and the substituent, and that the substitution results in a stable compound, *e.g.*, which does not spontaneously undergo transformation such as by rearrangement, cyclization, elimination, etc.

In some embodiments, the molecular weight of the acidic compound is below about 1000 g/mol. In some embodiments, the molecular weight of the acidic compound is between about 150 g/mol and about 1000 g/mol, *e.g*., between about 155 g/mol and about 750 g/mol, between about 160 g/mol and about 500 g/mol, between about 165 g/mol and about 250 g/mol, between about 170 g/mol and about 200 g/mol, or between about 175 g/mol and about 190 g/mol. In some embodiments, the molecular weight of the acidic compound is between about 180 g/mol and about 190 g/mol.

The acidic compound of Formula (I) may adopt any stereoisomeric form or contain a mixture of stereoisomers. For example, the acidic compound may be a mixture of D,L-phosphoserine, or contain substantially pure D-phosphoserine or substantially pure L-phosphoserine. In some embodiments, the stereochemistry of the acidic compound does not significantly impact the properties of the composition. In some embodiments, the particular stereochemistry of the compound of Formula (I) or the ratio of stereoisomers has a significant impact on the adhesive properties of the composition.

The amount of the acidic compound of Formula (I) may vary, *e.g.*, between 5% to 95% w/w of the total adhesive composition. In some embodiments, the amount of the acidic compound is in the range of 5% to about 80%, 5% to about 50%, 5% to about 30%, about 10% to about 80%, about 10% to about 50%, about 15% to about 40%, or about 20% to about 35% w/w of the total adhesive composition.

In the present disclosure, the multivalent metal salts (*e.g.*, calcium phosphates, calcium oxides, calcium hydroxides, or combinations thereof) react with the acidic compounds to form an adhesive composition when combined with an aqueous medium. In some embodiments, the aqueous medium comprises water (*e.g.*, sterile water), oral fluids (e.g., saliva, sulcular fluids, mucus, blood, or blood mixtures) buffers (*e.g.*, sodium phosphate, potassium phosphate, or saline), blood, blood-based solutions (*e.g*., plasma, serum, bone marrow), spinal fluid, dental pulp, cell-based solutions (*e.g*, solutions comprising fibroblasts, platelets, odontoblasts, erythrocytes, leukocytes, stem cells (*e.g.,* mesenchymal stem cells) histiocytes, macrophages, mast cells, or plasma cells), environmental water (*e.g.*, marine, fluvial or lacustrine (i.e., derived from the ocean or freshwater sources, *e.g.*, bays, lakes, streams, rivers, marshes, or ponds)), industrial process fluid, waste water (e.g., gray water or black water), or combinations thereof. In some embodiments, the aqueous medium is in the form of aqueous solutions, suspensions, or colloids. In some embodiments, the aqueous medium is water (e.g., liquid water). In some embodiments, the aqueous medium has an alkaline pH, a slightly aqueous pH, a neutral pH, a slightly acidic pH, or an acidic pH. In some embodiments, the aqueous medium is water (e.g., liquid water) and the pH of the water is neutral.

The components of the adhesive composition or device may be exposed to an aqueous environment through a mist, spray, injection, immersion or submersion.

In some embodiments, the compositions may further comprise an additive. In some embodiments, the compositions may further comprise a plurality of additives. An additive may be used to impart additional functionality to the composition of the disclosure, such as improving or affecting the handling, texture, durability, strength, porosity, or resorption rate of the material, or to provide additional cosmetic, conductive (*e.g.*, electrically or thermally conductive), medical, biological, or pharmacological properties. An additive may also be used to trap, hold, or adhere the components of the adhesive composition to the three dimensional fiber network material or mesh component or a solidified form of the adhesive composition prior to use of the device.

Exemplary additives may include a metal (*e.g*. copper, silver, gold, iron, titanium, aluminum, cobalt, chromium, tantalum), a metallic alloy (*e.g.* bronze, brass, stainless steel, cobalt-chromium), a salt comprising calcium, sodium, barium, strontium, lithium, potassium, magnesium, a phosphate, an oxide, hydroxide, iodide, a sulfate, a carbonate, fluoride, or chloride (*e.g.,* calcium phosphates (*e.g.,* dicalcium phosphate, monocalcium phosphate, beta tricalcium phosphate, hydroxyapatite, calcium depleted hydroxyapatites, alpha tricalcium phosphate), calcium oxide, calcium sulfate, calcium carbonate, calcium bicarbonate, calcium iodide, barium sulfate, barium phosphate, sodium phosphate, sodium carbonate, sodium bicarbonate, sodium chloride, magnesium phosphate, potassium chloride), a pore forming agent (*e.g*., through release of gas (e.g., calcium carbonate, sodium carbonate, sodium bicarbonate) or through dissolution of a solid (*e.g.,* sodium chloride, potassium chloride)), a humectant (*e.g*., sorbitol, or another hygroscopic compound), reducing or oxidizing agents, rust inhibitors, a polymeric alcohol (*e.g.,* polyethylene glycol), a filler, a formulation base, a viscosity modifier (*e.g.,* polyol (*e.g.,* glycerol, mannitol, sorbitol, trehalose, lactose, glucose, fructose, or sucrose)), an abrasive , a coloring agent (*e.g.,* a dye, pigment, or opacifier), a flavoring agent (*e.g.,* a sweetener), a medication that acts locally (*e.g.,* an anesthetic, coagulant, clotting factor, chemotactic agent, and an agent inducing phenotypic change in local cells or tissues), a medication that acts systemically (*e.g*., an analgesic, anticoagulant, hormone, vitamin, pain reliever, anti-inflammatory agent, chemotactic agent, or agent inducing phenotypic change in local cells or tissues), an antimicrobial agent (*e.g.,* an antibacterial, antiviral, antifungal agent), an antifouling agent (e.g., copper, silver, or other transition metal salts) or a combination thereof. In some embodiments, the additive comprises a polymer. The biologically active substances (*e.g.*, medicines, drugs) in the categories above might include active substances or precursors, which become biologically active upon modification after interaction with the surrounding environment. The biologically active substances might include a stem cell (*e.g.,* an adult, induced pluripotent, or mesenchymal stem cell) or bone tissue components (e.g., autograft, allograft, xenograft). The substances might be synthetic, semisynthetic, or biologically derived (*e.g.*, peptides, proteins, or small molecules). The substances might include, but not be limited to an anti-inflammatory compound (*e.g*., a steroid, nonsteroidal anti-inflammatory drug, or cyclooxygenase inhibitor), a complement protein, a bone morphogenic factor or protein, a hormone active locally or systemically (*e.g.*, parathyroid hormone, calcitonin, or prostaglandin), or other small molecule (*e.g.,* a calciferol, a secosteroid).

The solidified form of the adhesive composition is a product from the reaction of a multivalent metal salt and an acidic compound in an aqueous medium. In some embodiments, the solidified form of the adhesive composition comprises hydroxyapatite, tetracalcium phosphate, tricalcium phosphate, or any other calcium salt, e.g., as a byproduct from the reaction of a calcium phosphate metal salt, an acidic compound, and an additive or a plurality of additives in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, and poly(lactide-co-glycolide) fibers in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, poly(lactide-co-glycolide), and calcium carbonate in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, and a biologically active substance in an aqueous medium. In some embodiments, the solidified form of the adhesive composition comprises hydroxyapatite, tetracalcium phosphate, tricalcium phosphate, or any other calcium salt as a byproduct from the reaction of tetracalcium phosphate, phosphoserine, calcium carbonate, poly(lactide-co-glycolide), and a biologically active substance in an aqueous medium.

In some embodiments, the additive is a polymer. Suitable polymers incorporated as additives into the adhesive composition may contain functional groups comprising an electronegative atom as the bonding sites of the polymer surfaces to the available metal ions, such as a carbonyl oxygen atom (e.g., of an ester) or a nitrogen atom (e.g., of an amine) as the bonding sites of the polymer surfaces to the available metal ions. These functional groups can be either in the backbone chain of the polymer or in groups pendant to the polymer chain. These polymeric based compounds may include, but are not limited to, one or more of the following; poly(L-lactide), poly(D,L-lactide), polyglycolide, poly(ε-caprolactone), poly(teramethylglycolic-acid), poly(dioxanone), poly(hydroxybutyrate), poly(hydroxyvalerate), poly(lactide-co-glycolide), poly(glycolide-co-trimethylene carbonate), poly(glycolide-co-caprolactone), poly(glycolide-co-dioxanone-co-trimethylene-carbonate), poly(tetramethylglycolic-acid-co-dioxanone-co-trimethylenecarbonate), poly(glycolide-co-caprolactone-co-lactide-co-trimethylene-carbonate), poly(hydroxybutyrate-co-hydroxyvalerate), poly(methylmethacrylate), poly(acrylate), a polyamine, a polyamide, a polyimidazole, poly(vinyl-pyrrolidone), collagen, silk, chitosan, hyaluronic acid, collagen, gelatin and/or mixtures thereof. In addition, copolymers of the above homopolymers also can be used.

The general structural nature of a polymer (*e.g.*, a polymer used as an additive in an adhesive composition described herein) may include a linear homopolymer and copolymer, a cross linked polymer, a block polymer, a branched polymer, a hyper branched polymer, or a star shaped polymer. The polymers can be added to the formulation in the form of a solution, powder, fiber, resin, liquid crystal, hydrogel, chip, flake, and the like. The polymer can be included directly within the adhesive composition or can be an adjunct that is applied in situ as the adhesive composition is applied per its method of use (*e.g.,* to attach to bone).

An additive may be present in an amount from about 0.1% to about 99% weight by weight (w/w) of the total adhesive composition. In some embodiments, the additive or plurality of additives is present at a concentration of about 0.5%, about 1%, about 5%, about 10%, about 20%, about 30%, about 40%, about 50%, about 60%, about 70%, about 80%, about 90%, about 95%, or about 99% weight by weight (w/w) of the total adhesive composition.

In some embodiments, certain additives may be provided (i.e., prior to mixing with an aqueous medium) in the form as powders or granules or solutes or any combination thereof. These powders may exhibit a mean particle size of about 0.001 to about 0.750 mm, about 0.005 to about 0.150 mm, about 0.250 to about 0.750 mm, 0.25 to about 0.500, 0.015 to about 0.050 mm, about 0.015 to about 0.025 mm, about 0.020 to about 0.060 mm, about 0.020 to about 0.040 mm, about 0.040 to about 0.100 mm, about 0.040 to about 0.060 mm, about 0.060 to about 0.150 mm, or about 0.060 to about 0.125 mm. The mean particle size may be multi-modal (*e.g.*, bimodal) to include any combination of mean particle sizes as previously described. These granules may exhibit a mean granule size of about 0.025 mm to about 5 mm, about 0.100 to about 1.500 mm, about 0.125 to 1.000 mm, 0.125 to 0.500 mm, about 0.125 to 0.250 mm, about 0.250 to 0.750 mm, about 0.250 to 0.500 mm, about 0.500 to 1.00 mm, about 0.500 to 0.750 mm. The mean granule size may be multi-modal (*e.g.*, bimodal) to include any combination of mean granule sizes as previously described. In some embodiments, varying sizes of said powders or granules may be used in the adhesive composition.

In some embodiments, the granules may be roughly spheroidal or may be substantially flattened (e.g., oblate) or elongated (e.g., prolate) and have an aspect ratio of the equatorial semi-axis length to the axial semi-axis length in the range of about 100:1 to about 1:100, of about 100:1 to about 30:1, of about 50:1 to about 20:1, of about 30:1 to about 10:1, of about 20:1 to about 1:1, of about 1:1 to about 1:10, of about 1:5 to about 1:20, of about 1:10 to about 1:30, of about 1:20 to about 1:50, or of about 1:40 to about 1:100. The granules comprising the device may be uniform in shape or be a combination of two, or more differently shaped granules, depending on the application and the desired characteristics of the device. The granules comprising the device may be uniform in shape or be a combination of two, or more differently shaped and proportioned granules, depending on the application and the desired characteristics of the device. The granules comprising the device may be 100% roughly spherical or random roughly spheroidal in shape, or the device may be comprised of about 0.5-1% granules of different shape or aspect ratio, 1-2% granules of different shape or aspect ratio, 1.5-3% granules of different shape or aspect ratio, 2-4% granules of different shape or aspect ratio, 3-6% granules of different shape or aspect ratio, 4-8% granules of different shape or aspect ratio, 6-12% granules of different shape or aspect ratio, 10-20% granules of different shape or aspect ratio, 15-30% granules of different shape or aspect ratio, 20-40% granules of different shape or aspect ratio, 35-55% granules of different shape or aspect ratio, 50-75% granules of different shape or aspect ratio, 66-80% granules of different shape or aspect ratio, 75-90% granules of different shape or aspect ratio, 85-95% granules of different shape or aspect ratio, 90-95% granules of different shape or aspect ratio, 92-96% granules of different shape or aspect ratio, 95-99% granules of different shape or aspect ratio, or 100% granules of different shape or aspect ratio than roughly spheroidal in shape.

In some embodiments, certain additives may be provided (i.e., prior to mixing with an aqueous medium) as fibers. In some embodiments, the fibers may exhibit a mean diameter of about 0.010 mm to about 2 mm, about 0.010 mm to about 0.50 mm, or about 0.025 mm to about 0.075 mm. These fibers may exhibit a mean fiber length of about 0.025 mm to about 50.0 mm, about 0.50 mm to 10 mm, or about 1.00 mm to about 3.50 mm. The mean fiber diameter or length may be multi-modal to include any combination of mean fiber diameter or length as previously described.

In some embodiments, an additive may be supplied (i.e., prior to mixing with an aqueous medium) in the form of granules or fibers. In some embodiments, the additive is porous. In other embodiments, the additive is non-porous. In certain embodiments, the additive is porous and exhibits about 100% porosity. In certain embodiments, the additive is porous and exhibits about 98% porosity. In other embodiments, the additive is porous and exhibits about 10%, about 20%, about 30%, about 40%, about 50%, about 60%, about 70%, about 80%, about 90%, about 95%, or about 98% porosity.

In some embodiments wherein the additive is porous, the pores may be interconnected pores or closed pores. For example, in some embodiments, the mean diameter of the pores is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the mean diameter of the pores is from about 10 to about 500 nanometers, from about 25 to about 500 nanometers, from about 50 to about 500 nanometers, or from about 100 to about 500 nanometers.

In other embodiments, the mean diameter of the pores is in the micrometer range, e.g., from about 1 to about 1000 micrometers, from about 1 to about 750 micrometers, from about 1 to about 500 micrometers, from about 1 to about 250 micrometers, from about 1 to about 100 micrometers, or from about 1 to about 50 micrometers. In some embodiments, the mean diameter of the pores is from about 10 to about 500 micrometers, from about 25 to about 500 micrometers, from about 50 to about 500 micrometers, or from about 100 to about 500 micrometers.

In still other embodiments, the mean diameter of the pores is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the mean diameter of the pores is from about 1 to about 10 millimeters, from about 1 to about 7.5 millimeters, from about 1 to about 5 millimeters, or from about 1 to about 2.5 millimeters.

The device comprises a solidified form of an adhesive composition (e.g., granules) after the reaction has completed in the presence of an aqueous medium. In some embodiments, the mass ratio of the solidified form (e.g., granule) comprising the device relative to the mass of the additional layer (e.g., dry components of the adhesive composition) may be in the range from about 8:1 to about 1:5, from about 8:1 to about 5:1, from about 7:1 to about 4:1, from about 6:1 to about 3:1, from about 5:1 to about 2:1, from about 4:1 to about 1:1, from about 3:1 to about 1:1.5, from about 2:1 to about 1:1, from about 1:1 to about 1:1.5, from about 1.5:1 to about 1:3, or from about 1:2 to about 1:5. The solidified form (e.g., granules) may be mixed with unreacted adhesive composition powder components, which upon contact with the aqueous medium (moisture) are reacted into the adhesive form in the working state on the surface of the solidified form. Thus reacted mixture may then be applied. The ratio (w/w) of the dry component mass (e.g., granule and the adhesive composition powder) comprising the device relative the total mass of the aqueous medium may be in the range from about 5:1 to about 1:1, from about 4:1 to about 3.3:1, from about 3.5:1 to about 3:1, from about 3:1 to about 2:1, from about 2.5:1 to about 2:1, from about 2:1 to about 1.5:1, or from about 1.5:1 to about 1:1.

The adhesive composition adopts a pliable working state after mixing with an aqueous medium prior to hardening. In some embodiments, the pliable working state is present for up to about 30 minutes or less, depending on the components of said composition. In some embodiments, the adhesive composition adopts a pliable working state for less than or equal to about 30 minutes after mixing with an aqueous solution or suspension, e.g., less than about 20 minutes, less than about 15 minutes, less than about 10 minutes, less than about 5 minutes, less than about 3 minutes, less than about 2 minutes, less than about 1 minute, less than about 30 seconds, less than about 5 seconds after mixing with an aqueous solution or suspension. As described herein, the adhesive composition does not acquire substantial adhesive properties until mixed with an aqueous medium.

After a set amount of time, the adhesive composition adopts a hard, cement-like state. This process of conversion from the pliable working state to the cement-like state may be referred to as "hardening," "setting," or "curing." In some embodiments, the adhesive composition exhibits an adhesive strength in the cement-like state in the range of about 100 KPa to about 12,000 KPa, depending on the application and the particular components and ratios of components in said adhesive compositions. In some embodiments, the adhesive strength of the adhesive composition in the cement-like state is between about 100 KPa and e.g., about 10,000 KPa, about 9,000 KPa, about 8,000 KPa, about 7,000 KPa, about 6,000 KPa, about 5,000 KPa, about 4,000 KPa, about 3,000 KPa, about 2,000 KPa, about 1,000 KPa, about 750 KPa, about 500 KPa, about 250 KPa, or about 200 KPa. In some embodiments, the adhesive strength of the adhesive composition in the cement-like state is between about 100 KPa, about 200 KPa, about 300 KPa, about 400 KPa, about 500 KPa, about 600 KPa, about 700 KPa, about 800 KPa, about 900 KPa, about 1,000 KPa, about 2,500 KPa, about 5,000 KPa, about 7,500 KPa, about 10,000 KPa or about 12,000 KPa. In some embodiments, the adhesive strength of the adhesive composition in the cement-like state is in the range of about 200 KPa and about 2,500 KPa. In some embodiments, the particular components of the adhesive composition may be selected to achieve the desired strength depending on the intended use of the adhesive compositions. Specific components may be altered to achieve the desired adhesive properties of said composition based on the intended use or desired outcome.

The device may be comprised of greater than 1%, 5%, 10%, 20%, 30%, 40%, 50%, 60%, 70%, 80%, 90%, 95%, or more of the three dimensional fiber network material. In some embodiments, the three dimensional fiber network material comprises 10-90%, 20-80%, 25-75%, 30-70%, 40-60%, or 50% of the mass of the device. In other embodiments, the device may be comprised of greater than 1%, 5%, 10%, 20%, 30%, 40%, 50%, 60%, 70%, 80%, 90%, 95%, or more of the adhesive composition or components thereof. In some embodiments, the adhesive composition or components thereof comprises 10-90%, 20-80%, 25-75%, 30-70%, 40-60%, or 50% of the mass of the device.

In certain embodiments, the device is porous and exhibits about 98% porosity. In other embodiments, the device is porous and exhibits about 10%, about 20%, about 30%, about 40%, about 50%, about 60%, about 70%, about 80%, about 90%, about 95%, about 98%, about 99.9% or more porosity. In said embodiments, the device exhibits a porosity in the range of about 10-90%, about 15-50%, or about 20-40% porosity. Porosity may include elements of microporosity and macroporosity. In said embodiments, the device is substantially nonporous, or exhibits 99%, about 98%, about 95%, about 90%, about 80%, about 70%, about 60%, about 50%, about 40%, about 30%, about 20%, about 10%, about 5%, 1%, about 0.1% or less porosity. In an embodiment, the character of the porosity may change upon interaction with an aqueous medium. In an embodiment, the character of the porosity may change with the passage of time, upon interaction with the aqueous medium, or over the course of use of the device.

In some embodiments wherein the device is porous, the pores may be interconnected pores or closed pores. For example, in some embodiments, the mean diameter of the pores is in the nanometer range, e.g., from about 1 to about 1000 nanometers, from about 1 to about 750 nanometers, from about 1 to about 500 nanometers, from about 1 to about 250 nanometers, from about 1 to about 100 nanometers, or from about 1 to about 50 nanometers. In some embodiments, the mean diameter of the pores is from about 10 to about 500 nanometers, from about 25 to about 500 nanometers, from about 50 to about 500 nanometers, or from about 100 to about 500 nanometers.

In other embodiments, the mean diameter of the pores is in the micrometer range, e.g., from about 1 to about 1000 micrometers, from about 1 to about 750 micrometers, from about 1 to about 500 micrometers, from about 1 to about 250 micrometers, from about 1 to about 100 micrometers, or from about 1 to about 50 micrometers. In some embodiments, the mean diameter of the pores is from about 10 to about 500 micrometers, from about 25 to about 500 micrometers, from about 50 to about 500 micrometers, or from about 100 to about 500 micrometers.

In still other embodiments, the mean diameter of the pores is in the millimeter range, e.g., from about 1 to about 100 millimeters, from about 1 to about 75 millimeters, from about 1 to about 50 millimeters, from about 1 to about 25 millimeters, from about 1 to about 10 millimeters, or from about 1 to about 5 millimeters. In some embodiments, the mean diameter of the pores is from about 1 to about 10 millimeters, from about 1 to about 7.5 millimeters, from about 1 to about 5 millimeters, or from about 1 to about 2.5 millimeters.

In certain embodiments, the device comprises any three-dimensional object. Exemplary three-dimensional objects include those that are thin in profile (e.g., as is typical for a patch, plug, spacer, or tape-like material) or thick in profile (e.g., to form an object it may be replacing, such as a segment or whole body replacement of bone). In certain embodiments, the three-dimensional object comprises a solid core or a segment of the core removed, e.g., as in the case of a ring. The device may also be scalable, such that it may cover small areas (e.g., a micro-crack in an object) or large areas (e.g., gaps or holes resulting from a blast, explosion, or structural failure). The device may be trimmed, milled, or stretched to a custom size as needed for use. The device may comprise additional features (*e.g.*, notches, holes, grooves) for the passage of critical structures (*e.g.*, nerves, vessels, tendons, muscles).

In some embodiments, the device, comprising the solidified form of the adhesive composition, may comprise features and components intended to aid desirable processes and outcomes. For example, the structure may feature perforations to facilitate injection of the adhesive composition onto the recipient site; the structure may feature concavities or convexities (e.g., notches, grooves, ridges, bumps, reliefs) intended to allow greater contact with specific tissue (e.g., bone) or avoidance of contact with a tissue (e.g., muscle, tendon, or nerve); and the structure may comprise features intended to accelerate the rate of its interaction with the surrounding tissues (e.g., perforations, grooves , ridges) intended to increase its surface area and thereby its rate of biodegradation.

The device may be elastic or possess shape memory which allows it to rebound, contract, or swell to its intended form after application of a tensile load, torsional load to the device. Exemplary sources of a tensile or torsional load are manual manipulative forces, or are derived from the application of heat, light, or an aqueous environment. The heat might be applied from an external source to the device or the heat may be released through the interaction of the composition with the aqueous medium.

The device may further comprise a shaping component, which may form or hold the device into a particular shape or configuration prior to, during, and/or after application. In some embodiments, a shaping component is relatively stiff prior to its use but softens or dissolves under the application conditions. For example, a shaping component may be rigid prior to application of the device to hold the device in a desirable shape for application to a particular site of use, but may lose said stiffness upon activation of the device. A shaping component may comprise a water-soluble material (e.g., gelatin or starch). Use of a shaping component, wherein the shaping component loses its shape and stiffness upon activation, may allow the device to be manipulated for better use. A shaping component may be thermoplastic or may dissolve with an increase in temperature related to heat, e.g., the heat released during the interaction of the components of the adhesive composition with the aqueous medium.

In some embodiments, different variants of device may be packaged and marketed as a kit. For example, said kit may comprise separate containers, wherein a first container comprises the three dimensional fiber network material or the solidified form of the adhesive composition and a second container comprises the components of the adhesive composition. In such embodiments, the components of the adhesive composition may be separated into different containers. In some embodiments, said kits may comprise a container containing a multivalent metal salt (*e.g.*, calcium phosphates, calcium oxide, calcium hydroxide) with an acidic compound (*e.g.*, phosphoserine) present together and sealed under good packaging practices to preserve the shelf life of the individual components. If additives are included in said kit, they may be packaged within this container or within a separate container. The aqueous medium (*e.g.*, solution or suspension), if included, may be provided in a separate container. The kit may include additional components for the preparation or application of the adhesive devices, such as mixing bowls or surfaces, stirring sticks, spatulas, syringes, heat guns, or other preparation or delivery devices.

In other embodiments, the device is stored in a dry space (e.g., a vacuum sealed space, an air-tight space, a hermetically sealed space) to prevent the introduction of moisture and the activation of the adhesive composition prior to intended use. In some embodiments, the device is sterilized and stored in such a manner to prevent contamination with microbes or infectious agents prior to the intended use. For example, the device may be stored in a sealed package or envelope made ready to peel apart at the time of intended use.

In other embodiments, the solidified form of the device is stored in a wet or humid environment (e.g., packaging vessel filled with an aqueous medium or atmosphere) prior to use to preserve structural properties or to prepare the adhesive device in some manner for use. In some embodiments, the device is sterilized and stored in such a manner to prevent contamination with microbes or infectious agents prior to the intended use. For example, the device may be stored in a sealed package or envelope made ready to peel apart at the time of intended use.

### Methods of Using of Adhesive Devices

The present disclosure provides an adhesive composition for use in a method of joining separated bones, wherein the method comprises forming a device (e.g., a patch, plug, beam, plate, screw, rod, granule, spacer, cage, disc, tape device or other shape determined by the geometry or anatomy of the site of application) comprising a three dimensional fiber network material and a solidified form of the adhesive composition, as well as an additional layer of the adhesive composition in the working state coated or impregnated into or onto the surface of the device. Exemplary uses include treating or healing a subject suffering from a disease or condition.

Exemplary devices and uses thereof are depicted in FIGS. 1A-1C, FIGS. 2A-2C FIGS. 3A-3B, FIGS. 4A-4B, FIG. 5, FIGS. 6A-6C, FIGS. 7A-7C, FIGS. 8A-8B, FIGS. 9A-9B, FIGS. 10A-10B, FIG. 11A-11B, FIG. 12A-12B, FIG.13A-13B, FIG. 14, FIGS.15A-15B, FIG. 16, FIGS. 20A-20E, FIGS. 22A-22B and FIGS. 24A-24D. In one example, not claimed, a device, in the form of an adhesive patch comprises a three dimensional fiber network material (random orientation) mixed, dusted, coated, or impregnated with an adhesive composition, e.g., as shown in FIGS. 1A-1C. In one example, not claimed, the device, in the form of an adhesive patch comprises a three dimensional fiber network material (both random and parallel orientations) mixed, dusted, coated, or impregnated with an adhesive composition, e.g., as shown in FIGS. 2A-2C. In one example, not claimed, the device is applied along a surface-spanning defect in an object and may be used as a patch, e.g., as shown in FIGS. 3A-3B. In one example, not claimed, the device is applied along a surface-spanning crack between two objects in an object and may be used as a tape, e.g., as shown in FIGS. 4A-4B. In one embodiment, the device is applied as an interposition device between two objects and may be used as a patch or plug, e.g., as shown in FIG. 5. In one embodiment, the device is applied to the exterior (e.g., FIGS. 6A-6C) or interior (e.g., FIGS. 7A-7C) of the surface of a defect in or on an object and may be used as a patch or plug. In one embodiment, the device comprises layers connected through mechanical interlocking on one side of the three dimensional fiber network material, e.g., as shown in FIGS. 8A-8B. In one example, not claimed, the device is applied as a cylindrical cuff joining two separate segments of an object (e.g., a bone) and may be used as a patch or tape, e.g., as shown in FIGS. 9A-9B. In one embodiment, the device is substantially rigid in one dimension and substantially compliant in another (e.g., orthogonal) dimension and may be used as a patch or sheet, e.g., as shown in FIGS. 10A-10B. In one embodiment, the device comprises a solidified beam of the adhesive composition pre-reacted in an aqueous medium and a second layer of adhesive composition mixed in an aqueous medium and applied as a coating to the surface of the beam, e.g., as shown in FIGS. 11A-11B. In one embodiment, the device is applied between transverse processes of adjacent vertebral bodies as a beam with a second layer of adhesive composition coated on its surface and adhesively fixated between the transverse processes and along the *pars interarticularis,* e.g., as shown in FIGS. 12A-12B. In one embodiment, the device comprises a solidified disc of the adhesive composition pre-reacted in an aqueous medium and a second layer of adhesive composition mixed in an aqueous medium and applied as a coating to the top and bottom surfaces of the disc, e.g., as shown in FIGS. 13A-13B. In one embodiment, the device is applied between adjacent vertebral bodies as an interbody disc with a second layer of adhesive composition coated on its top and bottom surfaces and adhesively fixated between adjacent vertebral bodies, e.g., as shown in FIG. 14. In one embodiment, the device comprises a solidified rod of the adhesive composition pre-reacted in an aqueous medium and a second layer of adhesive composition mixed in an aqueous medium and applied as a coating to the surface of the rod, e.g., as shown in FIGS. 15A-15B. In one embodiment, the device is applied between adjacent vertebral bodies in the facet joint as a rod with a second layer of adhesive composition coated on its surface and adhesively fixated in the facet joint between adjacent vertebral bodies, e.g., as shown in FIG. 16. In one embodiment, the device is a solidified form of the adhesive composition, in the form of a beam (FIG. 20A), with optional modifications, rib features (FIG. 20B), reliefs for anatomical features on inferior surface (FIG. 20C), reliefs for anatomical features on both the inferior and lateral surfaces (FIG. 20D), or reliefs for anatomical features on both the inferior and lateral surfaces and injection ports for adhesive (FIG. 20E). In one embodiment, the device is a solidified form of the adhesive composition in the form of substantially uniform spherical granules with an additional layer of adhesive composition coating the granules (FIG. 22A) and a combination of spherical and rod-shaped granules with an additional layer of adhesive composition coating the granules and rods (FIG. 22B). In one embodiment, the device is a form of the adhesive composition in the form of a disc (FIG. 24A), with optional modifications, stabilizer extension (FIG. 24B), stabilizer extension with perforations and an injection port (FIG. 24C), primary stabilizer extension with perforations, secondary stabilizer extension, and an injection port (FIG. 24D).

In some embodiments, the device is a solidified form of the adhesive composition in the form of spherical granules (e.g., substantially uniform spherical granules, as in FIG. 22A) or a combination of spherical and rod-shaped granules, as in FIG. 22B, that conforms to the defect or gap upon placement. In the presence of an aqueous medium the device may gradually harden to form an open lattice that can be penetrated by tissue, fluids, and cells, and then vascularized. These spherical granules may be stored ready for use in a moist state (i.e., wet, hydrated or saturated with an aqueous medium), or they may be rendered wet or saturated with aqueous medium during preparation for their use. In some embodiments, the porosity between granules, e.g., granules connected to one another, allows for exchange of materials, such as tissue, fluids, and growth factors, as well as for rapid infiltration by cellular elements, neovascularization, and bone deposition while maintaining mechanical continuity across the gap.

In some embodiments, inclusion of rods with spheres enlarges the macro-porosity fraction. In some embodiments, grains of different composition are used, e.g., few percent one that may dissolve quickly (reasonably soluble needle shaped crystals like CaCO₃ or sulfate) and others that may maintain shape, size, or mechanical strength. In some embodiments, the volume ratio of granules is about 60% to about 80%.

In some embodiments, the method further comprises observation of a defect in an object (e.g., a wet, submerged, immersed, leaking, weeping, or oozing object) from which a fluid (e.g., an aqueous fluid) is emanating through a crack, fissure, breach or defect in the surface and a determination of the specific type of device (e.g., an adhesive device described herein) or the specific components thereof to utilize to seal or repair said object. The device of the present disclosure may be applied to an object in a number of ways. For example, in some embodiments, the method of application comprises placement on an object that is wet, submerged, immersed, leaking, weeping, or oozing from the surface. Exemplary crack, fissures, breaches or defects in a surface include, but are not limited to, perforations, ruptures, pores, pits, tears, corrosions, erosions, abrasions, microfractures, and the like. For example, a tear may comprise a tear in a blood vessel that is ruptured within the bone and is bleeding through a foramen or marrow space, or a tear or crack whereby cerebrospinal fluid leaks through the dura mater, a skull fracture, or other defect.

The surface to which the adhesive device is applied is bone. In some embodiments, the term "bone" may refer to the entire bone or to a specific layer of the bone, e.g., the cortical or hard bone, cancellous or spongy bone, or bone marrow. In one embodiment, the surface to which the device is applied comprises the cortical or hard bone. In one embodiment, the surface to which the device is applied comprises the cancellous or spongy bone. In one embodiment, the surface to which the device is applied comprises the bone marrow.

In some embodiments, the surface to which the device is applied may require a permanent application. In other embodiments, the surface to which the device is applied may require a rapid or temporary application.

In other embodiments, the method comprises use of the device as a splint through application of the device to the outer surface of an object or inner surface through a central shaft or canal of separated bone or bone fragments. In certain embodiments, the device is applied to one aspect of a separated object or multiple aspects of the separated object. The device may be used as a splint by wrapping or covering the area of the separated object near the fracture line (e.g., in case of long bones), or by inserting the device into the shaft or canal (e.g., in case of long bones).

The method comprises use of the device as an interposition between surfaces to be joined, e.g., by inserting the device within the separation line, or between the separated pieces of a body, or within the separation space between the bodies (*e.g.,* intervertebral bodies, facet joint). Upon exposure to an aqueous medium (e.g., an aqueous medium described herein), the device may solidify, adhere to, or seal the adjoining surfaces. The solidified device has structural strength to reinforce the damaged joint enabling load bearing properties. An exemplary use of the device at the interposition between two surfaces is the replacement of a disc that is removed between vertebral bodies in order to provide load-bearing structural strength fusion of vertebrae (e.g. spinal fusion). Other exemplary uses of the device at the interposition between two surfaces is the joining of two bone fragments together.

In other embodiments, the method further comprises use of an additional access device to aid in the application of the device to a surface or region which is difficult to reach or to which minimally invasive approaches are desired. Exemplary additional access devices to aid in such application include, but are not limited to, an access tube, cannula, guide, or pipe. In some embodiments, the device is threaded or pushed through the additional access device, e.g., through trimming, folding, twisting, or bending of the device. Once deployed at the intended site of application, the device may be unfolded, untwisted, or trimmed for use. In some embodiments, the device is introduced to its site of action by traction, such as by a suture, thread, or rope. In certain embodiments, the traction includes a suture affixed to a needle. In these cases, the suture may be wrapped around a structure and be used to pull the device into position on the far side of the structure.

The device, comprising a solidified form of the adhesive composition, may be formed in such a shape and size that it may span across, or fit within, a gap which is intended for repair, closure, obturation or filling (e.g., repair of non-loadbearing defect, e.g., cranial segmental resection, partial thickness resection of a long bone). The structure may be designed to conform to the defect size and shape (e.g., using CAT scan data, optical scan, direct impression, or other comparable methods) and fabricated using a 3D forming technique (e.g., binder jetting 3D printing, CAD/CAM milling, or other comparable methods). The surface of said structure is then rendered adhesive by coating with the adhesive composition. The surface is then placed into the defect, e.g., whereupon the composition is allowed to cure undisturbed and bond the structure to the recipient site, e.g., and where it is gradually replaced with bone.

The device, comprising a solidified form of the adhesive composition, may be formed in such a shape and size that it may span across a gap between two or more other structures, which might be mobile with respect to one another when a load is applied, which are to be adhesively joined by a second layer of the adhesive composition coated on said device with the intent to fixate, immobilize or join (e.g., spinal fusion, ankle fusion, fracture fixation, close gap after diaphysis full thickness bone resection.). The device is designed to conform to the defect size and shape (e.g., using CAT scan data, optical scan, direct impression, or other comparable method) and fabricated using a 3D forming technique (e.g., binder jetting 3D printing, CAD/CAM milling, or other comparable method). The surface of said structure is then rendered adhesive by coating with the adhesive composition. The surface is then placed across the gap, e.g., whereupon the composition is allowed to cure and bond the said structure to the recipient site, e.g., and where it is gradually replaced with bone.

In some embodiments, the adhesive composition is for use in a method to treat or heal a subject suffering from a disease or condition, such as cancer (*e.g.*, osteosarcoma), osteoporosis, rickets, osteogenesis imperfecta, fibrous dysplasia, Paget's disease of the bone, hearing loss, renal osteodystrophy, a malignancy of the bone, infection of the bone, severe and handicapping malocclusion, osteonecrosis, cleft palate, or other genetic or developmental disease. In some embodiments, the adhesive devices and methods used to repair a defect in a bone caused by a disease or condition, such as cancer (*e.g.,* osteosarcoma), osteoporosis, rickets, osteogenesis imperfecta, fibrous dysplasia, Paget's disease of the bone, hearing loss, renal osteodystrophy, a malignancy of the bone, infection of the bone, or other genetic or developmental disease. In some embodiments, the devices and methods are used to strengthen a bone in a subject that has been weakened by a disease or condition, such as cancer (*e.g.*, osteosarcoma), osteoporosis, rickets, osteogenesis imperfecta, fibrous dysplasia, Paget's disease of the bone, hearing loss, renal osteodystrophy, a malignancy of the bone, infection of the bone, or other genetic or developmental disease. In some embodiments, the subject has experienced a trauma, such as a broken bone, fractured bone, or damaged tooth. In some embodiments, the subject has experienced tooth decay.

In some embodiments, the device may be packaged and stored ready for use in a wet state or saturated state with an aqueous medium, or it may be rendered moist (wet, hydrated or saturated with aqueous medium) during preparation for use (in which case the optimal amount of aqueous medium may be premeasured and packaged separately but with the structure within the kit). The surface of the said structure may be coated with unreacted adhesive composition powder components during preparation for application (also packaged within the kit either separately of together with the structure), which upon contact with the aqueous medium are reacted into the adhesive form. The reacted adhesive composition on the surface may then be used to attach the structure across or into the gap. Alternatively, the powder and liquid components may be each packed separately within the kit and mixed prior to contact with the structure.

### Methods of Fabricating Devices

The device (e.g., a patch, plug, beam, plate, screw, rod, granule, spacer, cage, disc, tape device, or other shape determined by the geometry or anatomy of the site of application) comprises a solidified form of the adhesive composition and a three dimensional fiber network, and further comprises a second layer of the adhesive composition in the working state, coated or impregnated into or onto the surface of the adhesive device.

In certain instances, fabrication of the device may comprise the following steps: (1) dissolving or suspending a three dimensional fiber network material in a solvent resulting in formation of a solution; (2) suspending the components of an adhesive composition in the solution or suspension prepared in step (1); (3) suspending other solid particles into the suspension formed in step (2); (4) mixing the suspension formed in step (3); (5) fully or partially filling or casting the suspension formed in step (4) into a mold or container that defines the outer shape of a device; (6) removing (e.g., selectively removing) the solvent of the suspension of step (5) through evaporation, which may be enhanced by partial vacuum or application of heat to recover or reconstitute the solid device comprised of the components of the adhesive composition of step (2) and the other solid particles of step (3) interspersed in a matrix of the three dimension fiber network material; and/or (7) removing (e.g., selectively removing) the other solid particles of step (3) to produce a porous adhesive device.

In some embodiments, the solvent used in formation of a solution outlined in step (1) comprises chloroform, dichloromethane, acetone, benzene, tetrahydrofuran, diethyl ether, or dimethyl sulfoxide, provided the components of the adhesive composition (e.g., of step (2)) and the other solid particles (e.g., of step (3)) are substantially insoluble in said solvent.

In some embodiments, the other solid particles (e.g., of step (3)) comprise an additive. The mean particle size of said additive may be within a range from about 10 microns to about 1000 microns, from about 20 microns to about 750 microns, from about 25 microns to about 500 microns, or from about 50 microns to about 250 microns. The mean particle size of said additive may also be within a range from about 500 microns to about 5000 microns, or from about 500 microns to about 2000 microns.

In some embodiments, the other solid particles (e.g., of step (3), e.g., an additive) are added such that the percentage mass of the other solid particles to the mass of the any one of the three dimensional fiber network material, the components of the adhesive composition, and the solvent may be in the range of about 5% m/m, about 10% m/m, about 15% m/m, about 20% m/m, about 25% m/m, about 30% m/m, about 40% m/m, about 50% m/m, about 60% m/m, about 70% m/m, about 80% m/m, or more. In certain embodiments, the percentage mass of the other solid particles to any one of the three dimensional fiber network material, the components of the adhesive composition, and the solvent is from about 10 % to about 90% m/m, from about 10% to about 50% m/m, or from about 10% to about 30% m/m.

The other solid particles (e.g., of step (3), e.g., the additive) may be removed from the adhesive device prior to packaging of said device. In certain embodiments, the other solid particles may be removed from the adhesive device prior to packaging of the device by exposure and dissolution in a solvent, wherein both the three dimensional fiber network material and the components of the adhesive composition are substantially insoluble in said solvent, and wherein said solvent does not substantially initiate the reaction of the components of the adhesive composition. In certain embodiments, the solvent is substantially pure (e.g. about 100%, about 99.9%, about 99.5%, about 95%, about 90%, or about 70% pure). In some embodiments, the solvent comprises an impurity, e.g., water. In certain embodiments, the solvent is isopropyl alcohol. The removal of said solvent may be through passive evaporation or evaporation enhanced by partial vacuum or heating. In some embodiments, the other solid particles comprise glucose, mannitol, or sorbitol.

In certain embodiments, the removal of other solid particles (e.g., of step (3), e.g., the additive) from the device occurs during application of the device, during the period following the application of the device, or at another time.

The removal of other solid particles (e.g., of step (3), e.g., the additive) from the device may occur through sublimation. The sublimation may involve addition of heat to the system, and/or application of vacuum (e.g., partial vacuum) to the system. In certain embodiments, the other solid particles comprise solid particles of iodine, carbon dioxide (e.g., dry ice), or water (e.g., ice).

The removal of other solid particles (e.g., of step (3), e.g., the additive) from the device may occur during application of the device or in the period following application of the device through exposure and dissolution of the device in a solvent, wherein the three dimensional fiber network material is substantially soluble or insoluble in said solvent and said solvent is a medium capable of activating the reaction of the components of the adhesive composition. In certain embodiments, the solvent is an aqueous medium. In some embodiments, the shape of the container which defines the final form of the device is developed taking into account diagnostic or radiographic data. In other embodiments, the shape of the container which defines the final form of the device is customized (e.g., milled or cut to shape) prior to packaging or by the end user.

Various methods of fabrication of the device may comprise deposition of components of the adhesive composition onto or into the three dimensional fiber network material or into or onto the mesh component through a process comprising dusting, trapping through filtration, adhering, creation of an electrostatic interaction, capillarity, centrifugation, rocking, agitation, vibration, or partial activation of particular components of the adhesive composition by introduction of a polar, charged, or neutral agent. In certain embodiments, an additive such as an adhesive (e.g. rubber adhesive) could be first introduced to the three dimensional fiber network material or into or onto the surface of the mesh component through filtration or by solvent evaporation in order to subsequently trap, hold, or adhere the adhesive composition components that are applied by filtration, dusting, or other means to the three dimensional fiber network material or into or onto the surface of the mesh component. In certain embodiments, the partial activation comprises addition of small or well-defined quantities of an aqueous medium, or other polar, charged or neutral agent so as to allow partial reaction (e.g. setting, or curing) of the components of the adhesive composition to the elements of the three dimensional fiber network material or mesh component, while retaining capacity for complete or full reaction of the adhesive composition components later during use of the device. The method of using an electrostatic interaction to deposit the adhesive composition components onto or into the three dimensional fiber network material or the mesh component may comprise electrostatically charging the three dimensional fiber network material or the mesh component with one polarity which is opposite from the polarity of the components of the adhesive composition.

Methods of fabrication of the device may comprise deposition of the components of the adhesive composition into the three dimensional fiber network material through suspension of the components in a fluid medium applied to the structural component of the device. The resulting suspension may be applied as a slurry. **In** certain cases, centrifugation, vibration or other means of agitation may be used to aid dispersal and mixing of the components of the adhesive composition within the framework of the three dimensional fiber network material.

The fluid medium may comprise a substantially nonpolar component (e.g., cyclopentane, hexanes, or dichloromethane), or a component substantially incapable of forming hydrogen bonds (e.g., chloroform or benzene). The fluid medium may comprise a substantially non-ionizable component (e.g., tetrahydrofuran), or a volatile solvent (e.g., chloroform, dichloromethane, acetone, tetrahydrofuran, diethyl ether, acetone, ethanol, or isopropyl alcohol). The fluid medium may provide an environment in which the components of the adhesive composition are substantially insoluble. **In** certain embodiments, the fluid medium does not significantly initiate the reaction of the components of the adhesive composition. **In** certain embodiments, the fluid medium comprises a primary component present in a defined percentage (e.g. about 100%, about 99.9%, about 99.5%, about 95%, about 90%, or about 70%), and a secondary component present in a defined percentage (e.g. about 0.1%, about 0.5%, about 1.0%, about 5.0%, about 10%, or about 30%). The secondary component may partially activate the adhesion composition components. **In** some embodiments, the secondary component comprises a solvent for the components of the adhesive composition (e.g. ethanol, methanol, water). **In** some embodiments, the primary component of the fluid medium is isopropyl alcohol and the secondary component of the fluid medium is water. The removal of said solvent may be through passive evaporation or evaporation enhanced by partial vacuum or heating.

**In** certain embodiments, the fluid medium is characterized by low surface tension and by low solubility of the three dimensional fiber network material. The fluid medium may further be removed through wicking, passive evaporation or evaporation enhanced by partial vacuum or application of heat.

Methods of storage of the device may comprise maintenance of the solid components of the adhesive composition within the three dimensional fiber network material or into or onto the surface of the mesh component by coating the device with said components through compression, vacuum forming, or partial activation of the particular components of the adhesive composition by introduction of a polar, charged, or neutral agent, or through introduction of an aqueous medium (e.g., an aqueous medium described herein). **In** certain embodiments, the three dimensional fiber network material or the mesh component may be retained or stable in the presence of the components of the adhesive composition or the activated adhesive device. For example, maintenance of the components of the adhesive composition within the three dimensional fiber network or the surface of the mesh component could be achieved by coating said components of the adhesive composition and retaining three dimensional fiber network or the mesh component after deposition. **In** other embodiments, the three dimensional fiber network material or the mesh component may be dissolved or removed after deposition of the components of the adhesive composition, or after activation of the device. For example, the three dimensional fiber network or mesh component may be soluble in the aqueous medium or permeable to the aqueous medium.

**In** certain embodiments, the partial activation of particular components of the adhesive composition comprises addition of small or well-defined quantities of the aqueous medium, or other polar, charged or neutral agent. **In** certain embodiments, maintenance or storage of the components of the adhesive composition within the three dimensional fiber network material or into or onto the surface of the mesh component is achieved using defined quantities of an adhesive (e.g. rubber adhesive) deposited as a coating to hold, trap, or adhere the components of the adhesive composition to the three dimensional fiber network material or into or onto the surface of the mesh component.

**In** some embodiments, the method of stabilization or retention of the components of the adhesive composition within the three dimensional fiber network or the mesh component after deposition may be achieved by inclusion of a small, well-defined amount of a stabilizing component. The stabilizing component may be ionizable. The stabilizing component may interact with the components of the adhesive composition. The stabilizing component may be polymeric. An exemplary stabilizing component is Carbomer^{®} material.

**In** certain embodiments, the method of fabrication of the device comprises methods of fabrication of the surface of the three dimensional fiber network material or the mesh component. **In** some embodiments, the three dimensional fiber network material comprises an ionizable surface. This ionizable surface may be produced through incorporation of a precursor monomer as copolymer elements, which may comprise a reactive chemical group such as a carboxyl, nitrile, amine , halide, acyl halide, organometal, aryl, sulfonyl, or another chemical group that might be ionizable or capable of conversion to a positively or negatively charged species.

**In** certain embodiments, the precursor monomer co-polymerizes with base monomers through an addition reaction. The precursor monomer may co-polymerize with the base monomers through reaction of another reactive chemical compound, e.g., a compound comprising a butadiene, acrylonitrile, styrene, acrylic acid, vinyl chloride, or a derivative thereof (e.g., an acrylic acid ester, e.g., methyl methacrylate, ethyl methacrylate, etc.), which may be added in amounts appropriate to obtain desired properties. The precursor monomer may co-polymerize through an ester linkage (e.g., caprolactone) or through an amide linkage (e.g., caprolactam).

**In** certain embodiments, the ionizable surface element is produced through the addition of a reactive chemical group to the base polymer through formation of a chemical bond. The chemical bond may involve formation of an ester, amide, ether, disulfide, or other linkage type. The chemical bond may be covalent or non-covalent in nature, or an ionic bond (e.g., an ionomer).

The ionizable surface element may comprise any of a variety of different chemical functionalities. **In** certain embodiments, the ionizable surface element results from a reaction of a base polymer group with a reagent, for example, wherein a substitution reaction to the aromatic side groups of styrene polymers and copolymers is carried out in the presence of sulfuric acid to yield sulfonyl groups (e.g., bound to the para position of said aromatic side groups) bearing a negative charge. **In** some embodiments, the ionizable surface element results from a reaction of a base polymer group with a reagent, for example, wherein reduction of a nitrile group within acrylonitrile polymers and copolymers yields an amine bearing a positive net charge. **In** some embodiments, the ionizable surface element results from a reaction of a base polymer group with a reagent, for example, wherein reduction of a nitrile group within acrylonitrile polymers and copolymers with lithium aluminum hydride results in formation of a primary amine bearing a positive net charge. **In** some embodiments, the ionizable surface element results from a reaction of a base polymer group with a reagent, for example, wherein reaction of a nitrile group within acrylonitrile polymers and copolymers with a Grignard reagent to yield a carbonyl group allows for further opportunities for installation of additional grafting and attachment of prosthetic groups

The processes which will determine the ultimate surface characteristics of the structural elements may occur at any point of the fabrication of the elements. For example, these processes may be carried out during the formation of the three dimensional fiber network of the device, prior to the deposition or loading of the components of the adhesive composition, after the deposition of the adhesive composition, during the activation of the device at the time of the activation of the adhesive composition, through the introduction of an aqueous medium, or at any other time.

**In** other embodiments, the method of fabrication of the device further comprises methods of adhering a mesh component to the interconnected fiber network. **In** certain embodiments, the mesh component is applied to the three dimensional fiber network through spraying or pouring the pre-cursor polymer components, resulting in entrapping the fiber network and interlocking the two layers through polymerization. **In** other embodiments, the mesh component is applied to the three dimensional fiber network material through spray or pouring of a molten thermoplastic polymer component, resulting in entrapping the fiber network interlocking the two layers during the cooling phase.

**In** certain embodiments, the mesh component is metallic. The metallic mesh component may be applied to the three dimensional fiber network through a mechanism that involves a phase change (e.g. sintering, spot welding, or ultrasonic welding) of the metallic mesh component, resulting in entrapping the fiber network and interlocking the two layers through polymerization. The metallic component may comprise solid metal elements, e.g., plates, foils, or scales.

Fabricating the adhesive device in the form of a patch, plug, beam, plate, screw, rod, granule, spacer, cage, disc, tape device, or other shape determined by the geometry or anatomy of the site of application, wherein the device comprises a solidified form of the adhesive composition (e.g., pre-reacted in an aqueous medium),may comprise the following steps: (1) preparing a mixture of powders (e.g., adhesive composition powders) and optionally adding an additive; (2) adding an aqueous medium to the mixture of powders from step (1) to form an adhesive composition; (3) fully or partially filling or casting the adhesive composition of step (3) into a mold or container or onto an outer surface that defines the outer shape of a device; (4) allowing the adhesive composition to solidify; (5) reshaping (e.g., selectively reshaping) the device to incorporate a geometric feature (e.g. a hole, threads, or tunnel) into or onto the device, and/or milling the device to a desired powder or granule size; and (6) impregnating or coating the device with an adhesive composition.

In some embodiments, the device is prepared through a three-dimensional printing method (e.g., binder jetting).

In some embodiments, the device is subjected to sterilization at any point of the fabrication process, e.g., before packaging, during an intermediate packaging stage, or prior to or after final packaging. Sterilization methods may comprise filtration; exposure to ethylene oxide, gamma irradiation, or e-beam irradiation; steam sterilization through use of wet or dry techniques; or a combination thereof.

### EXAMPLES

Some embodiments presented herein are further described in detail by reference to the following examples. These examples are provided for purposes of illustration and are not intended to be limiting unless otherwise specified. The disclosure should be construed to encompass any and all variations which become evident as a result of the teaching provided herein.

Without further description, it is believed that one of ordinary skill in the art can, using the preceding description and the following illustrative examples, make and utilize the compounds and practice the claimed methods. The following examples specifically point out various aspects of the disclosure, and are not to be construed as limiting in any way the remainder of the disclosure.

### Example 1: Adhesive Compositions

Exemplary adhesive compositions are specified in Table 1. The solid components listed in the table may be supplied in particle, granule, or fiber form, and the size of each of the components listed in Table 1 may range as described in the Detailed Description. In some embodiments, the resulting properties such as working and setting time is be affected by these changes. The specific mean particle, granule, or fiber size for each solid component was selected to satisfy the use requirements as described in each of the embodiments.

The quantities of each of the components listed may be altered or adjusted in relation to the other components in the composition. After mixing, the compositions described were applied to the desired site and the adhesive properties examined, e.g., for tensile strength and durability. Each composition may further comprise phases and ingredients not indicated in the table.

**Table 1: Components of Exemplary Adhesive Compositions**

| | **Composition** | | | | | | |
|---|---|---|---|---|---|---|---|
| **Component** | **A** | **B** | **C** | **D** | **E** | **F** | **G** |
| Tetracalcium phosphate | 800 mg | 800 mg | 800 mg | 0 | 800 mg | 800 mg | 800 mg |
| Phosphoserine | 500 mg | 500 mg | 500 mg | 500 mg | 500 mg | 500 mg | 500mg |
| Hydroxyapatite | 0 | 0 | 0 | 0 | 0 | 375 mg | 375 mg |
| Alpha Tricalcium Phosphate | 0 | 375 mg | 0 | 675 mg | 0 | 0 | 0 |
| Beta Tricalcium Phosphate | 0 | 0 | 375 mg | 0 | 0 | 0 | 0 |
| Calcium Carbonate | 0 | 0 | 0 | 125 mg | 200 mg | 0 | 0 |
| Sorbitol | 0 | 0 | 0 | 0 | 0 | 0 | 0 |
| Poly(lactide-co-glycolide) | 0 | 0 | 0 | 0 | 0 | 0 | 50 mg |

### Reference Example 2: Spinal Fixation and Fusion - Inter-Transverse Process, Rabbit

Exemplary Device A is a solidified form of the adhesive composition, in the form of a beam, and produced by mixing the solid components of Adhesive Composition G described in Example 1 with 325 µl of water using a spatula for 20 to 30 seconds in a 25 ml silicone mixing vessel to form a homogenous adhesive slurry. The adhesive slurry was back filled into a 3 ml syringe and the plunger was inserted to gather and inject the adhesive slurry into a PTFE mold channel defined by three orthogonal walls of a cavity (i.e., base and two sides). The dimensions of the channel were 6 mm in width *(i.e.,* distance between the surfaces of the side walls) x 4 mm in height *(i.e.,* distance from the top surface of the open cavity down to the surface of the base wall) x 25 mm in length (distance from end to end of the open cavity). The dimensions were defined to produce a rectangular beam matching the cavity dimensions. After filling the mold with the adhesive slurry while it was still within the working period, a spatula was used to smooth and level the adhesive slurry to match the contour of the cavity and whereby excess material was removed. The adhesive slurry was allowed to cure for 10 minutes at room temperature conditions into a solidified beam after which the solidified beam was removed from the mold cavity. The beam was placed end to end to contact the decorticated surfaces of both the L5 and L6 transverse processes and along the *pars interarticularis* region. Thereafter, a second layer of Adhesive Composition G described in Example 1 was prepared with 325 µl of water as described previously and injected to coat the beam where in contact with the decorticated surfaces of both the L5 and L6 transverse processes and along the *pars interarticularis* region to comprise a second layer. After 3 minutes of curing, the second layer of Adhesive Composition G solidified forming an adhesive bond between the beam and the transverse processes and the *interarticularis* region. Device A served as a solid bridge construct between the L5/L6 transverse processes thereby providing fixation to substantially immobilize the L5 and L6 vertebrae (i.e. spine fusion). This procedure was repeated on the contralateral side with a second application of Device A as described here to form bilateral fixation of the L5/L6 transverse processes.

The procedure described above was repeated on several rabbits to assess healing and long term maintenance of the fixation. Following a time course of 0, 6, 16, 31 or 39 weeks of post-surgical intervention, the rabbits were radiographed by cone beam computer tomography (CBCT) (as shown in FIGS. 17A-17E) and euthanized. Dorsolateral view of three-dimensional reconstructions of the CBCT data at immediately post-surgical intervention and at 39 weeks of post-surgical intervention (FIG. 17F-17G). The L4/L5/L6 interbody segments were harvested from the spine. The intervertebral discs between the L4/L5 vertebral body and between the L5/L6 vertebral body were severed to detach their connection along with any ligaments that formed a connection between these vertebral body segments. Care was taken not to disrupt the bilateral fixation formed by Device A between the L5/L6 vertebral segments. The L5/L6 vertebral bodies were mounted onto an axial force testing machine (i.e. Instron 5969) using a pin inserted through each vertebral body and wires that formed a connection to the Instron as shown in FIG. 18. Following this mounting procedure, a tensile load was applied until failure. Likewise, the adjacent L4/L5 vertebral bodies, which served as an untreated control site, were mounted onto the axial force testing machine and a tensile load was applied until failure. The tensile strength results from this testing following a time course of 0, 6, 10, and 20 weeks of post-surgical intervention are presented in FIG. 19. In this figure, the tensile load to failure shows a 1.4x, 2.8x, 4.0x, and 9.1x average increase in relative strength between the L5/L6 vertebral bodies treated with Device A and the untreated L4/L5 vertebral body control sites as at 3, 6, 10 and 20 weeks respectively.

### Reference Example 3: Three dimensional fiber network Material Impregnated with an Adhesive Composition

Exemplary Device C comprises a three dimensional fiber network material impregnated with an adhesive composition in the form of an adhesive patch, produced by the following steps: (1) dissolution or suspension of 1g poly(lactide-co-glycolide) in 3.5ml chloroform resulting in formation of a solution; (2) suspension of the components of the Adhesive Composition A described in Example 1in the solution prepared in step (1); mixing of the suspension resulting from step (2); (3) fully filling the suspension into a mold that defined the outer shape of the rectangular patch (34mm length x 8mm width x 2mm thickness); (4) removal of the chloroform by evaporation in an oven set to 40°C to reconstitute the solid form of Device C comprised of Adhesive Composition A impregnated in a three dimensional fiber network of poly(lactide-co-glycolide).

Device C was tested for bone-bone adhesive tensile strength. The bone blocks used for adhesive testing were prepared from a bovine femur cortical source. Each cube used for testing was rectangular with dimensions of 8.5 mm x 8.5 mm x 20 mm. On one end of each of the bone blocks there was a 2mm through hole used to mount the blocks for adhesive testing. The bone blocks were stored at -20° C prior to testing to preserve their structure. Prior to testing, the bone blocks were removed from the freezer and preconditioned by submerging them in a phosphate buffered saline (PBS) bath at 37° C for at least one hour. Immediately prior to testing, the bone blocks were removed from the bath, excess aqueous solution was not removed from the surface *(i.e.,* the surfaces were wet), and they were aligned end to end such that that 8.5 mm x 8.5 mm faces opposite the mounting holes made contact. Device C was submerged in a container filled with 25ml water for 10 seconds and applied as a patch which wrapped the ends of the two blocks forming a joint which adhered the two bone blocks together. Thereafter, the adhered blocks were submerged into a PBS bath at 37° C to allow Device C to cure for t=10 minutes from the time of patch application. After the cure time indicated, the adhered blocks were removed from the PBS bath for adhesive testing. The adhered bone blocks were tested on an Instron^{®} 5969 axial load frame by securing the adhered blocks, using 1.8mm mounting pins inserted through the mounting holes of each cube, to mounting fixtures located on both the fixed surface and the crosshead of the Instron. The test was run with the crosshead speed at 2mm/minute. The tensile load to failure was 105N.

### Example 4: Solidified Form of Adhesive Composition in the Form of Porous Granules

An exemplary Device D is a solidified form of the adhesive composition, in the form of porous granules, produced by mixing a multiple of 5 times the solid components of Adhesive Composition E described in Example 1 with 3 ml of water using a spatula for 20 to 30 seconds in a 25 ml silicone mixing vessel to form a homogenous adhesive slurry. The mixing vessel holding the adhesive slurry was gently tapped to allow the material to settle and form a level surface within. The adhesive slurry was allowed to cure for 10 minutes at room temperature before being transferred to a humidity chamber set to 50-65°C for an additional 15 - 60 minutes. The solidified adhesive device was removed as a porous block from the mixing bowl and broken into several wedges using an Arbor press. The wedges of material were processed through a jaw crusher (i.e., Reutsch Model#BB50) first through a 5 mm gap setting. The material was collected and re-processed again through the jaw crusher, but with a 2 mm gap setting. The collected material was next processed through a co-mill (Quattro Model#193) selected to reduce the porous granule size to the desired size range. In this example, the material was co-milled at 750 rpm through a stainless screen with mesh size of 4,750 µm and a spacer of 0.3". The porous granules were collected again and re-processed through the co-mill through a similar fashion, but with a series of reduced mesh size stainless steel screens ranging from larger mesh (2,388 µm) run at 750 rpm to smaller mesh (610 µm) run at 2,000 rpm. The granules were collected and sieved between stainless screens of 105 µm to 250 µm. The granules were packed for storage to protect the material from moisture contamination. These granules can be utilized as an additive in the adhesive compositions disclosed or as a standalone component or ingredient of other devices. Device D produced in this example was used as an additive comprising hydroxyapatite outlined in Adhesive Composition F and Adhesive Composition G described in Example 1.

### Example 5: Spinal Fixation - Inter-Transverse Process and Inter Articular Process, Cadaveric Sheep

Exemplary Device E is a solidified form of the adhesive composition, in the form of a beam, comprising additional layers of the adhesive composition in its working (i.e., when pliable and tacky) state as a coating on the surface of the beam with the function of adhering it to bone. The beam component is produced by mixing a 12x dose of Adhesive Composition G described in Example 1 with 1650 µl of water using a spatula for 20 to 30 seconds in a 25 ml silicone mixing vessel to form a homogenous adhesive slurry. The adhesive slurry was transferred into a PTFE mold channel defined by a lower base plate with a non-stick membrane, PTFE mold that creates the walls of a cavity followed by an upper PTFE compression insert and upper pressure plate used to compress the material as it sets into the designed shape and size. The dimensions of the channel were 13.2 mm in width *(i.e.,* distance between the surfaces of the side walls) x 6.3 mm in height *(i.e.,* distance from the top surface of the open cavity down to the surface of the base wall) x 60 mm in length (distance from end to end of the open cavity). The dimensions were defined to produce a parallelepiped beam matching the cavity dimensions. The PTFE mold was placed onto an aluminum base plate with a non-stick membrane sandwiched between the mold and the base plate. The now-captured membrane was locked into position when the two parts (mold and base plate) were attached using a series of clearance and tapped holes that were tightened to create an enclosed cavity. Using a spatula, the mold was filled with the adhesive slurry while the adhesive was still in working state. The PTFE compression insert was placed into the mold cavity and compressed to begin forming the material into the design shape. The upper platen was then mounted and secured to the filled mold assembly/compression insert until the upper plate was in planar contact with the PTFE mold. The hardware was tightened, then the mold assembly was placed into a water bath and allowed to cure for thirty minutes. Once cured, the upper and lower plate were removed. Using the compression insert, firm pressure was used to release the cured material from the mold. The compression insert and newly formed beam were removed from the mold cavity as one. A razor blade was used to release the molded beam from the compression insert and to trim flashing. A detail sander was used to remove any sharp edges.

The preparation of the transverse processes included stripping of the periosteum, decortication of the dorsal surface, and pulsed lavage treatment. The beam was applied to the dorsal decorticated surfaces of the L5 and L6 transverse processes and along the *pars interarticularis* region. The beam shape was modified by using an abrasive detail sander, or dremel tool to accommodate the geometry of the attachment site (FIG. 20C). Thereafter, a 6X dose of Adhesive Composition G described in Example 1 was prepared with 825µl of water as before and loaded into a 3ml syringe. The syringe was then used to inject the mixed material onto the beam where it would contact the decorticated surfaces of the L5 and L6 transverse processes to form a second, adhesive layer to fixate beam, which was then placed onto the decorticated attachment sites and pressed gently into position. Excess adhesive material was removed within the allotted working time. After 3 minutes of curing, an additional 6X dose of Adhesive Composition G described in Example 1 was prepared with 825µl of water as before and loaded into a 3ml syringe. This material was injected to form an additional layer or layers making contact between the beam and the *pars interarticularis* and to encapsulate the decorticated and pulse lavaged articular processes. Following 3 minutes of curing, the adhesive solidified forming a bond between Device E and the transverse processes, *interarticularis* region, and the articular processes (FIG. 21A). The now-fixated spine section was allowed to complete curing in a water bath at 37°C for thirty minutes.

This procedure was repeated on the contralateral side with a second application of Device E as described here to form bilateral fixation at the L5/L6 transverse processes, *interarticularis* region, and articular processes. Upon completion, the assembly was allowed to fully cure in a water bath at 37°C for thirty minutes. Device E served as a solid bridge construct between the L5 and L6 transverse processes with encapsulation of the decorticated and pulse lavaged articular processes thereby providing fixation of the L5/L6 vertebral bodies. This is equivalent to the operative procedure leading to spine fusion *in vivo.*

The L5/L6 vertebral bodies were explanted, debrided, and mounted onto an axial force testing machine (i.e., Instron 5969) using a pin inserted through each vertebral body and wires that formed a connection to the testing machine (FIG. 21B). Following this mounting procedure, a tensile load was applied until failure. The results of this test show results of >248N.

### Example 6: Spinal Fixation - Inter-Articular Process, Human Cadaver

Exemplary Device F is a solidified form of the adhesive composition, in the form of a disc, comprising an additional layer of the adhesive composition in its working (i.e., when pliable and tacky) state as a coating on the surface of the disc with the function of adhering it to bone (FIG. 24A). The disc was produced by mixing a multiple of 5 times the solid components of Adhesive Composition G described in Example 1 with 1,625 µl of water using a spatula for 20 to 30 seconds in a 25 ml silicone mixing vessel to form a homogenous adhesive slurry. The adhesive slurry was loaded from the breech into a 5ml syringe, and the plunger was inserted to gather and inject the adhesive slurry into a PTFE mold defined by a cylindrical cavity. The dimensions of the cavity were 25mm in diameter x 6 mm in depth *(i.e.,* distance from the top surface of the open cavity down to the surface of the base). The dimensions were defined to produce a cylindrical disc matching the cavity dimensions. After filling the mold with the adhesive slurry while pliable, a spatula was used to smooth and level the adhesive slurry to match the contour of the cavity and remove excess material. The adhesive slurry was allowed to cure for 10 minutes at room temperature conditions into a solidified disc after which the solidified disc was removed from the mold cavity. The dorsal extent of the articular processes from L5 and L6 was removed to reveal a pair of flattened stub surfaces on each articular process. Each newly exposed surface was decorticated, and cleaned using the pulsed lavage techniques. Device F, the disc, was placed to cover the decorticated surfaces of both the L5 and L6 vertebral bodies. A second layer of Adhesive Composition G described in Example 1 was prepared with 325 µl of water as before and injected to coat the disc surface where in contact with the decorticated surfaces of both the L5 and L6 articular processes. Thereafter the disc was placed onto the newly prepared attachment sites and gentle pressure was applied to lock the discs onto the bone surfaces. Excess material was remove during the working time. After 3 minutes of curing the second layer of Adhesive Composition G solidified to form a bond between the disc and the articular processes. Device F served as a solid bridge construct between the L5/L6 articular surfaces thereby providing fixation to relatively immobilize the L5 and L6 vertebrae (FIG. 23).

### Reference Example 7: Fracture Fixation, Extra-osseous Internal Splint - Metatarsal Diaphysis, Oblique, Cadaveric Sheep

Exemplary Device G comprises a three dimensional fiber network material coated or impregnated with an adhesive composition in the form of an adhesive tape (or patch), produced by mixing the solid components of Adhesive Composition F described in Example 1 with 325 µl of water using a spatula for 20 to 30 seconds in a 25 ml silicone mixing vessel to form a homogenous adhesive slurry. The adhesive slurry was coated or impregnated over an interconnected fiber matrix of size 20 mm in width, 80 mm in length and 3 mm in thickness. The tape was then wrapped around an oblique fracture experimentally created in the diaphysis of a sheep metatarsal (FIG. 4B) to function as an internal splint after curing. After wrapping, the adhesive was allowed to cure by holding it in slight compression for 4 minutes before being placed in a PBS bath at 37° C to allow Device G to cure to 30 minutes. Thereafter, the adhered metatarsal was placed within an axial load testing machine (i.e., Instron^{®} 5969) by securing the proximal metatarsal in a custom potting fixture and placing the distal segment against a platen, holding the hoof in full contact to the platen. A compressive force, at 20° relative the long axis of the bone, was then applied to the proximal metatarsal segment with a crosshead speed of 5mm/minute. The compressive/shear load to failure was 57 kg (125 pounds).

## Claims

1. An adhesive composition for use in a method of joining separated bones, wherein the method comprises:
(1) forming a device for j oining the bones, wherein the device comprises:
(a) a solidified form of the adhesive composition and a three dimensional fiber network, wherein the adhesive composition comprises a multivalent metal salt and an acidic compound and the acidic compound comprises:
a compound of Formula (I) or a salt thereof 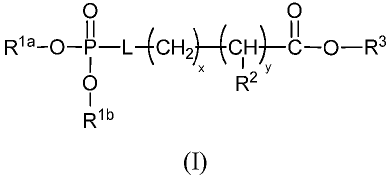 wherein
L is O, S, NH, or CH₂;
each of R^{1a} and R^{1b} is independently H, optionally substituted alkyl, or optionally substituted aryl;
R² is H, NR^{4a}R^{4b}, C(O)R⁵, or C(O)OR⁵;
R³ is H, optionally substituted alkyl, or optionally substituted aryl;
each of R^{4a} and R^{4b} is independently H, C(O)R⁶, or optionally substituted alkyl;
R⁵ is H, optionally substituted alkyl, or optionally substituted aryl;
R⁶ is optionally substituted alkyl or optionally substituted aryl; and
each of x and y is independently 0, 1, 2, or 3; and
(b) an additional layer of the adhesive composition in the working state present as a coating on a surface of the device or impregnated into or onto a surface of the device, wherein the working state of the adhesive composition is a pliable state adopted after mixing with an aqueous medium and prior to hardening, and the solidified form of the adhesive composition is formed after the reaction between the multivalent metal salt and the acidic compound has completed in the presence of an aqueous medium, and
(2) applying the device at the interposition between the two separated bones.

2. The adhesive composition for use of claim 1, wherein the device is in the form of a patch, plug, beam, plate, screw, rod, granule, spacer, cage, disc, tape device, or other shape determined by the geometry or anatomy of the site of application.

3. The adhesive composition for use of claim 1 or claim 2, wherein the adhesive composition further comprises an additive or plurality of additives.

4. The adhesive composition for use of claim 3, wherein the additive or plurality of additives comprises a pore forming agent.

5. The adhesive composition for use of any one of claims 1-4, wherein the three dimensional fiber network material is in the form of a two or three-dimensional grid, lattice, mesh, mat, weave, braid, cloth, fabric, felt, web, open cell foam, sponge, sheet, membrane, cage, or gel.

6. The adhesive composition for use of any one of claims 1-5, wherein the mean diameter of the three dimensional fiber network material is from 1 micrometer to 100 micrometers, or from 100 micrometers to 500 micrometers, or from 500 micrometers to 1000 micrometers.

7. The adhesive composition for use of any one of claims 1-6, wherein the device is trimmed, milled, or stretched to a custom size.

8. The adhesive composition for use of any one of claims 1-7, wherein the multivalent metal salt comprises tetracalcium phosphate or tricalcium phosphate.

9. The adhesive composition for use of claim 1, wherein the three dimensional fiber network comprises a substantially biocompatible or substantially bioresorbable component.

10. The adhesive composition for use of claim 9, wherein the biocompatible or bioresorbable component comprises calcium-based components, glass-ceramic or bioactive glass components, or one or more biopolymers.

11. The adhesive composition for use of claim 1, wherein the device exhibits a porosity in the range of 1-99%.

12. The adhesive composition for use of claim 1, wherein the three dimensional fiber network comprises a substantially non-resorbable component.

13. The adhesive composition for use of claim 1, wherein the three dimensional fiber network comprises a mesh component, optionally wherein the three dimensional fiber network comprises elements, threads, or components that are structurally interconnected or intertwined.

14. The adhesive composition for use of claim 1, wherein the amount of the acidic compound of Formula (I) comprises between 5% to 95% w/w of the total adhesive composition.

15. The adhesive composition for use of claim 1, wherein the device is comprised of greater than 1% of the three dimensional fiber network material.

## Patentansprüche

1. Klebstoffzusammensetzung zur Verwendung in einem Verfahren zum Verbinden getrennter Knochen, wobei das Verfahren Folgendes umfasst:
(1) Bilden einer Vorrichtung zum Verbinden der Knochen, wobei die Vorrichtung Folgendes umfasst:
(a) eine verfestigte Form der Klebstoffzusammensetzung und eines dreidimensionalen Fasernetzwerks, wobei die Klebstoffzusammensetzung ein mehrwertiges Metallsalz und eine saure Verbindung umfasst und die saure Verbindung Folgendes umfasst:
eine Verbindung der Formel (I) oder ein Salz davon, 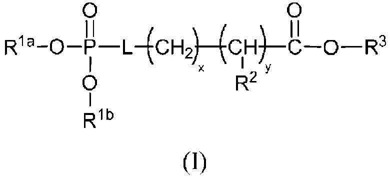 wobei
L für O, S, NH oder CH₂ steht;
R^{1a} und R^{1b} jeweils unabhängig für H, gegebenenfalls substituiertes Alkyl oder gegebenenfalls substituiertes Aryl stehen;
R² für H, NR^{4a}R^{4b}, C(O)R⁵ oder C(O)OR⁵ steht;
R³ für H, gegebenenfalls substituiertes Alkyl oder gegebenenfalls substituiertes Aryl steht;
R^{4a} und R^{4b} jeweils unabhängig für H, C(O)R⁶ oder gegebenenfalls substituiertes Alkyl stehen;
R⁵ für H, gegebenenfalls substituiertes Alkyl oder gegebenenfalls substituiertes Aryl steht;
R⁶ für gegebenenfalls substituiertes Alkyl oder gegebenenfalls substituiertes Aryl steht; und
x und y jeweils unabhängig für 0, 1, 2 oder 3 stehen; und
(b) eine zusätzliche Schicht der Klebstoffzusammensetzung im Arbeitszustand, die als Beschichtung auf einer Oberfläche der Vorrichtung vorliegt oder in eine Oberfläche der Vorrichtung oder auf dieser imprägniert ist, wobei der Arbeitszustand der Klebstoffzusammensetzung ein biegsamer Zustand ist, der nach Mischen mit einem wässrigen Medium und vor Härten angenommen wird, und die erstarrte Form der Klebstoffzusammensetzung gebildet wird, nachdem die Reaktion zwischen dem mehrwertigen Metallsalz und der sauren Verbindung in Gegenwart eines wässrigen Mediums abgeschlossen ist, und
(2) Anwenden der Vorrichtung an der Zwischenposition zwischen den beiden getrennten Knochen.

2. Klebstoffzusammensetzung zur Verwendung nach Anspruch 1, wobei die Vorrichtung in Form eines Pflasters, eines Stopfens, einer Schiene, einer Platte, einer Schraube, eines Stabs, eines Granulats, eines Abstandshalters, eines Käfigs, einer Scheibe, einer Bandvorrichtung oder einer anderen Form vorliegt, die durch die Geometrie oder Anatomie des Anwendungsorts bestimmt wird.

3. Klebstoffzusammensetzung zur Verwendung nach Anspruch 1 oder 2, wobei die Klebstoffzusammensetzung ferner ein Additiv oder mehrere Additive umfasst.

4. Klebstoffzusammensetzung zur Verwendung nach Anspruch 3, wobei das Additiv oder die mehreren Additive ein porenbildendes Mittel umfassen.

5. Klebstoffzusammensetzung zur Verwendung nach einem der Ansprüche 1 bis 4, wobei das dreidimensionale Fasernetzwerkmaterial in Form eines zwei- oder dreidimensionalen Rasters, eines Gitters, eines Netzes, einer Matte, eines Gewebes, eines Geflechts, eines Tuchs, eines Stoffs, eines Filzes, einer Gewebebahn, eines offenporigen Schaums, eines Schwamms, einer Folie, einer Membran, eines Käfigs oder eines Gels vorliegt.

6. Klebstoffzusammensetzung zur Verwendung nach einem der Ansprüche 1 bis 5, wobei der mittlere Durchmesser des dreidimensionalen Fasernetzwerkmaterials von 1 Mikrometer bis 100 Mikrometer oder von 100 Mikrometer bis 500 Mikrometer oder von 500 Mikrometer bis 1000 Mikrometer beträgt.

7. Klebstoffzusammensetzung zur Verwendung nach einem der Ansprüche 1 bis 6, wobei die Vorrichtung auf eine benutzerdefinierte Größe zugeschnitten, gemahlen oder gestreckt wird.

8. Klebstoffzusammensetzung zur Verwendung nach einem der Ansprüche 1 bis 7, wobei das mehrwertige Metallsalz Tetracalciumphosphat oder Tricalciumphosphat umfasst.

9. Klebstoffzusammensetzung zur Verwendung nach Anspruch 1, wobei das dreidimensionale Fasernetzwerk eine im Wesentlichen biokompatible oder im Wesentlichen bioresorbierbare Komponente umfasst.

10. Klebstoffzusammensetzung zur Verwendung nach Anspruch 9, wobei die biokompatible oder bioresorbierbare Komponente Komponenten auf Calciumbasis, Glaskeramik- oder bioaktive Glaskomponenten oder ein oder mehrere Biopolymere umfasst.

11. Klebstoffzusammensetzung zur Verwendung nach Anspruch 1, wobei die Vorrichtung eine Porosität im Bereich von 1 bis 99 % zeigt.

12. Klebstoffzusammensetzung zur Verwendung nach Anspruch 1, wobei das dreidimensionale Fasernetzwerk eine im Wesentlichen nicht resorbierbare Komponente umfasst.

13. Klebstoffzusammensetzung zur Verwendung nach Anspruch 1, wobei das dreidimensionale Fasernetzwerk eine Maschenkomponente umfasst, wobei das dreidimensionale Fasernetzwerk gegebenenfalls Elemente, Fäden oder Komponenten umfasst, die strukturell miteinander verbunden oder miteinander verflochten sind.

14. Klebstoffzusammensetzung zur Verwendung nach Anspruch 1, wobei die Menge der sauren Verbindung der Formel (I) zwischen 5 % und 95 Gew.-%, bezogen auf die gesamte Klebstoffzusammensetzung, umfasst.

15. Klebstoffzusammensetzung zur Verwendung nach Anspruch 1, wobei die Vorrichtung mehr als 1 % des dreidimensionalen Fasernetzwerkmaterials umfasst.

## Revendications

1. Composition adhésive à utiliser dans un procédé d'assemblage d'os disjoints, le procédé comprenant :
(1) la formation d'un dispositif destiné à assembler les os, le dispositif comprenant :
(a) une forme solidifiée de la composition adhésive et un réseau tridimensionnel de fibres, la composition adhésive comprenant un sel métallique multivalent et un composé acide et le composé acide comprenant :
un composé de formule (I) ou un sel de celui-ci 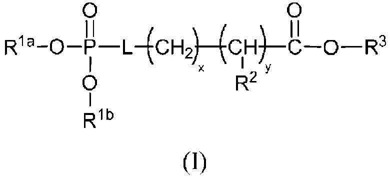
dans lequel
L représente O, S, NH, ou CH₂ ;
R^{1a} et R^{1b} représentent chacun indépendamment H, un alkyle éventuellement substitué, ou un aryle éventuellement substitué ;
R² représente H, NR^{4a}R^{4b}, C(O)R⁵, ou C(O)OR⁵ ;
R³ représente H, un alkyle éventuellement substitué, ou un aryle éventuellement substitué ;
R^{4a} et R^{4b} représentent chacun indépendamment H, C(O)R⁶, ou un alkyle éventuellement substitué ;
R⁵ représente H, un alkyle éventuellement substitué, ou un aryle éventuellement substitué ;
R⁶ représente un alkyle éventuellement substitué ou un aryle éventuellement substitué ; et
x et y valent chacun indépendamment 0, 1, 2, ou 3 ; et
(b) une couche supplémentaire de la composition adhésive à l'état de mise en œuvre présente sous forme de revêtement sur une surface du dispositif ou imprégnée dans ou sur une surface du dispositif, l'état de mise en œuvre de la composition adhésive étant un état pliable adopté après mélange avec un milieu aqueux et avant durcissement, et la forme solidifiée de la composition adhésive étant formée une fois que la réaction entre le sel métallique multivalent et le composé acide s'est achevée en présence d'un milieu aqueux, et
(2) l'application du dispositif à l'interface entre les deux os disjoints.

2. Composition adhésive pour une utilisation de la revendication 1, le dispositif prenant la forme d'une pièce, d'un bouchon, d'une barre, d'une plaque, d'une vis, d'une tige, d'un granulé, d'une entretoise, d'une cage, d'un disque, d'un dispositif en ruban, ou une autre forme déterminée par la géométrie ou l'anatomie du site d'application.

3. Composition adhésive pour une utilisation de la revendication 1 ou la revendication 2, la composition adhésive comprenant en outre un additif ou une pluralité d'additifs.

4. Composition adhésive pour une utilisation de la revendication 3, l'additif ou la pluralité d'additifs comprenant un agent porogène.

5. Composition adhésive pour une utilisation de l'une quelconque des revendications 1 à 4, le matériau en réseau tridimensionnel de fibres se présentant sous la forme d'une grille, d'un treillis, d'un maillage, d'une natte, d'une armure, d'une tresse, d'une étoffe, d'un tissu, d'un feutre, d'une bande, d'une mousse à cellules ouvertes, d'une éponge, d'une feuille, d'une membrane, d'une cage ou d'un gel, bi- ou tridimensionnels.

6. Composition adhésive pour une utilisation de l'une quelconque des revendications 1 à 5, le diamètre moyen du matériau en réseau tridimensionnel de fibres étant de 1 micromètre à 100 micromètres, ou de 100 micromètres à 500 micromètres, ou de 500 micromètres à 1000 micromètres.

7. Composition adhésive pour une utilisation de l'une quelconque des revendications 1 à 6, le dispositif étant découpé, usiné ou étiré à une taille sur mesure.

8. Composition adhésive pour une utilisation de l'une quelconque des revendications 1 à 7, le sel métallique multivalent comprenant du phosphate tétracalcique ou du phosphate tricalcique.

9. Composition adhésive pour une utilisation de la revendication 1, le réseau tridimensionnel de fibres comprenant un composant sensiblement biocompatible ou sensiblement biorésorbable.

10. Composition adhésive pour une utilisation de la revendication 9, le composant biocompatible ou biorésorbable comprenant des composants à base de calcium, des composants en vitrocéramique ou en verre bioactif, ou un ou plusieurs biopolymères.

11. Composition adhésive pour une utilisation de la revendication 1, le dispositif présentant une porosité comprise entre 1 et 99 %.

12. Composition adhésive pour une utilisation de la revendication 1, le réseau tridimensionnel de fibres comprenant un composant sensiblement non résorbable.

13. Composition adhésive pour une utilisation de la revendication 1, le réseau tridimensionnel de fibres comprenant un composant maillé, le réseau tridimensionnel de fibres comprenant éventuellement des éléments, fils ou composants qui sont structuralement interconnectés ou entrelacés.

14. Composition adhésive pour une utilisation de la revendication 1, la quantité du composé acide de formule (I) représentant entre 5 % et 95 % pds/pds de la composition adhésive totale.

15. Composition adhésive pour une utilisation de la revendication 1, le dispositif étant composé de plus de 1 % du matériau en réseau tridimensionnel de fibres.
